# EUROPEAN PATENT APPLICATION

(11) **EP 4 342 427 A1**
(43) Date of publication of application: **27.03.2024**
(21) Application number: 23193874.7
(22) Date of filing: 29.08.2023
(51) Int. Cl.: A61F 2/24

(54) **TRANSCATHETER HEART VALVE PROTHESIS AND METHOD OF ASSEMBLING**

(30) Priority: 01.09.2022 US 202263403132 P; 21.07.2023 US 202318356729
(71) Applicant: Medtronic, Inc., Minneapolis, Minnesota 55432 (US)
(72) Inventor: KIBRIA, Alkindi, Santa Ana (US); OLNEY, Karl L., Santa Ana (US); GARDE, Kshitija P., Santa Ana (US); TRAN, Kelly T., Santa Ana (US); LEUNG, Philip C., Santa Ana (US); LUU, Ashlee A., Santa Ana (US); CHESHKO, Tasha, Santa Ana (US); WOEN, Veronica, Santa Ana (US); CASLEY, Mark, Santa Rosa (US); KIM, Ambar A., Irvine (US); DINH, Thuy Linh, Santa Ana (US)
(74) Representative: Zimmermann & Partner Patentanwälte mbB

(57) **Abstract**

A transcatheter heart valve prosthesis and a method of assembling the transcatheter heart valve prosthesis are disclosed. The heart valve prosthesis includes a valve-skirt assembly having an inner skirt, a frame, an outer wrap backing and an outer wrap. The method includes: tacking the valve-skirt assembly within the frame; attaching the inner skirt below commissure posts of the frame; attaching commissures of the valve component to the commissure posts; attaching a plurality of outer wrap backings to the inner skirt; attaching the outer wrap backings and the inner skirt to the frame; attaching tissue bumpers to struts in an outflow section of the frame; attaching an outer wrap and the inner skirt to the frame; attaching the outer wrap to the inner skirt proximate inflow edges thereof; and attaching the outer wrap to the inner skirt and to the outer wrap backings proximate respective outflow edges thereof.

## Description

### FIELD OF THE INVENTION

The present invention generally relates to medical devices. More particularly, the present technology is related to a transcatheter heart valve prosthesis and a method of assembling the transcatheter heart valve prosthesis.

### BACKGROUND

The human heart is a four chambered, muscular organ that provides blood circulation through the body during a cardiac cycle. The four main chambers include the right atrium and right ventricle which supplies the pulmonary circulation, and the left atrium and left ventricle which supplies oxygenated blood received from the lungs into systemic circulation. To ensure that blood flows in one direction through the heart, atrioventricular valves (tricuspid and mitral valves) are present between the junctions of the atrium and the ventricles, and semi-lunar valves (pulmonary valve and aortic valve) govern the exits of the ventricles leading to the lungs and the rest of the body. These valves contain leaflets or cusps that open and shut in response to blood pressure changes caused by the contraction and relaxation of the heart chambers. The valve leaflets move apart from each other to open and allow blood to flow downstream of the valve, and coapt to close and prevent backflow or regurgitation in an upstream manner.

Diseases associated with heart valves, such as those caused by damage or a defect, can include stenosis and valvular insufficiency or regurgitation. For example, valvular stenosis causes the valve to become narrowed and hardened which can prevent blood flow to a downstream heart chamber from occurring at the proper flow rate and may cause the heart to work harder to pump the blood through the diseased valve. Valvular insufficiency or regurgitation occurs when the valve does not close completely, allowing blood to flow backwards, thereby causing the heart to be less efficient. A diseased or damaged valve, which can be congenital, age-related, drug-induced, or in some instances, caused by infection, can result in an enlarged, thickened heart that loses elasticity and efficiency. Some symptoms of heart valve diseases can include weakness, shortness of breath, dizziness, fainting, palpitations, anemia and edema, and blood clots which can increase the likelihood of stroke or pulmonary embolism. Symptoms can often be severe enough to be debilitating and/or life threatening.

Heart valve prostheses have been developed for repair and replacement of diseased and/or damaged heart valves. Such heart valve prostheses can be percutaneously delivered and deployed at the site of the diseased heart valve through catheter-based delivery systems. Such heart valve prostheses are delivered in a radially compressed or crimped configuration so that the heart valve prosthesis can be advanced through the patient's vasculature. Once positioned at the treatment site, the heart valve prosthesis is expanded to engage tissue at the diseased heart valve region to, for instance, hold the heart valve prosthesis in position.

The present disclosure relates to methods of assembling a heart valve prosthesis. More particularly, the present invention relates to methods of assembling a transcatheter heart valve prosthesis having a stent-like frame, a valve component, an inner skirt, an outer wrap backing and an outer wrap that together provide a reduced profile. The transcatheter heart valve prosthesis further includes an unsupported margin of attachment (MOA) below each leaflet of a valve component that allow the load to transfer from the leaflet to the skirt material to reduce the risk of leaflet tearing. Additionally, in cases of non-uniform deployment, the MOA is less distorted because the skirt can accommodate for some of the slack or tension due to asymmetry.

### BRIEF SUMMARY OF THE INVENTION

In accordance with a first example hereof, a transcatheter valve prosthesis includes a frame having an inflow section and an outflow section, the frame being a substantially tubular structure that defines a central lumen of the prosthesis, a valve skirt assembly having a valve component and an inner skirt, the valve-skirt assembly being disposed within the central lumen of the frame, an outer wrap backing attached to an exterior of the inflow section of the frame, and an outer wrap disposed over the outer wrap backing and attached to the frame to cover the remainder of the exterior of the inflow section of the frame.

In a second example, in a valve prosthesis of the first example or any of the subsequent examples herein, the inner skirt has a scalloped outflow edge that is comprised of a plurality of cut-outs and a plurality of peak regions.

In a third example, in a valve prosthesis according to any of the previous or subsequent examples herein, each cut-out extends between adjacent peak regions of the inner skirt.

In a fourth example, in a valve prosthesis according to any of the previous or subsequent examples herein, the outer wrap backing is a plurality of outer wrap backings with each outer wrap backing of the plurality of outer wrap backings being disposed on the exterior of the frame opposite a respective cut-out of the plurality of cut-outs of the inner skirt.

In a fifth example, in a valve prosthesis according to any of the previous or subsequent examples herein, each of the outer wrap backing has a plurality of tabs extending from an outflow edge thereof that are attached to a plurality of struts of a top strut row of the inflow section of the frame.

In a sixth example, in a valve prosthesis according to any of the previous or subsequent examples herein, the valve component is comprised of a plurality of leaflets with each leaflet of the plurality of leaflets being attached to the inner skirt at a respective margin of attachment.

In a seventh example, in a valve prosthesis according to any of the previous or subsequent examples herein, bottom curved portions of the margin of attachments between the plurality of leaflets and the inner skirt are not directly attached to the frame.

In an eighth example, in a valve prosthesis according to any of the previous or subsequent examples herein, the outer wrap backing and the outer wrap are made of biocompatible materials that prevent or prohibit paravalvular leakage through the frame of the valve prosthesis.

In a ninth example, in a valve prosthesis according to any of the previous or subsequent examples herein, straight inflow edges of the inner skirt and the outer wrap are aligned with each and the straight inflow edges are spaced from inflow nodes of an inflow node row of the frame to leave the inflow nodes exposed.

In a tenth example, in a valve prosthesis according to any of the previous or subsequent examples herein, the inflow edges of the inner skirt and the outer wrap are attached to each other by a line of horizontal stitches that parallels the inflow edges without wrapping around the frame therebetween.

In an eleventh example, a method of assembling a transcatheter heart valve prosthesis includes tacking a valve-skirt assembly within an interior of a frame, the valve skirt assembly having a valve component and an inner skirt, attaching the inner skirt to the frame within a plurality of inferior commissure areas that are located below a plurality of commissure posts of the frame, attaching a plurality of commissures of the valve component to the plurality of commissure posts of the frame, attaching a plurality of outer wrap backings to the inner skirt with the frame situated therebetween, attaching the plurality of outer wrap backings and the inner skirt to the frame, attaching tissue bumpers to struts proximate to or within an outflow section of the frame, attaching an outer wrap and the inner skirt to the frame, attaching the outer wrap to the inner skirt proximate inflow edges thereof, and attaching the outer wrap to the inner skirt and to the outer wrap backings proximate outflow edges thereof.

In a twelfth example, in a method of the eleventh example or any of the subsequent examples herein, each outer wrap backing of the plurality of outer wrap backings is disposed on the exterior of the frame opposite a respective cut-out of a plurality of cut-outs within the outflow edge of the inner skirt.

In a thirteenth example, in a method according to any of the previous or subsequent examples herein, attaching the plurality of outer wrap backings to the inner skirt includes securing a curved outflow edge of each of the outer wrap backings to the inner skirt, proximate a respective cut-out, by using a suture to create a blanket-stitch pattern therebetween.

In a fourteenth example, in a method according to any of the previous or subsequent examples herein, attaching the plurality of outer wrap backings and the inner skirt to the frame includes attaching a plurality of tabs that extend from the outflow edge of each outer wrap backing to a plurality of struts of a top strut row of an inflow section of the frame.

In a fifteenth example, in a method according to any of the previous or subsequent examples herein, attaching the outer wrap and the inner skirt to the frame includes attaching the inflow edge of the outer wrap to a plurality of struts of a bottom strut row of an inflow section of the frame by using a suture to create a wrap-frame attachment pattern proximate the inflow edge of the outer wrap that secures the outer wrap to the plurality of struts of the bottom strut row.

In a sixteenth example, in a method according to any of the previous or subsequent examples herein, attaching the outer wrap to the inner skirt proximate inflow edges thereof includes aligning the inflow edges of the inner skirt and the outer wrap with each such that the inflow edges are spaced from inflow nodes of an inflow node row of the frame to leave the inflow nodes exposed.

In a seventeenth example, in a method according to any of the previous or subsequent examples herein, attaching the outer wrap to the inner skirt proximate inflow edges thereof includes using a suture to create a line of horizontal stitches that parallels the inflow edges of the outer wrap and the inner skirt without the suture wrapping around the frame therebetween.

In an eighteenth example, in a method according to any of the previous or subsequent examples herein, attaching the outer wrap to the inner skirt and to the outer wrap backings proximate outflow edges thereof includes using a suture to create a line of outflow edge stitches that parallels struts in a third strut row of the frame without the suture wrapping around the frame.

In a nineteenth example, in a method according to any of the previous or subsequent examples herein, the valve component is comprised of a plurality of leaflets with each leaflet of the plurality of leaflets being attached to the inner skirt at a respective margin of attachment.

In a twentieth example, in a method according to any of the previous examples, bottom curved portions of the margin of attachments between the plurality of leaflets and the inner skirt are not directly attached to the frame.

In a twenty-first example, a transcatheter valve prosthesis includes a frame defining a central lumen of the prosthesis, a valve skirt assembly having a valve component and an inner skirt, the valve-skirt assembly being disposed within the central lumen of the frame, wherein the inner skirt has a scalloped edge comprised of a plurality of cut-outs that are disposed against an interior of the frame, and a plurality of outer wrap backings with each outer wrap backing of the plurality of outer wrap backings being disposed on the exterior of the frame opposite a respective cut-out of the plurality of cut-outs of the inner skirt.

In a twenty-second example, in the valve prosthesis of the twenty-first example, further includes an outer wrap disposed over the plurality of outer wrap backings and over at least a portion of a remainder of the exterior of the frame.

In a twenty-third example, a transcatheter valve prosthesis includes a frame defining a central lumen of the prosthesis, an inner skirt disposed within the central lumen against an interior of the frame, and an outer wrap disposed over at least a portion of an exterior of the frame, wherein inflow edges of the inner skirt and the outer wrap are aligned and co-extensible with each other and spaced a defined distance from an inflow node row of the frame such that inflow crowns of the inflow node row are exposed, and wherein the inflow edges of the inner skirt and the outer wrap are attached to each other without being attached to the frame therebetween such that the inflow edges of the inner skirt and the outer wrap are readily slidable on a first strut row of the frame when the prosthesis is expanded.

Further disclosed herein is a transcatheter heart valve prosthesis and a method of assembling the transcatheter heart valve prosthesis are disclosed, wherein the heart valve prosthesis includes a valve-skirt assembly having an inner skirt, a frame, an outer wrap backing and an outer wrap, and wherein the method includes: tacking the valve-skirt assembly within the frame; attaching the inner skirt below commissure posts of the frame; attaching commissures of the valve component to the commissure posts; attaching a plurality of outer wrap backings to the inner skirt; attaching the outer wrap backings and the inner skirt to the frame; attaching tissue bumpers to struts in an outflow section of the frame; attaching an outer wrap and the inner skirt to the frame; attaching the outer wrap to the inner skirt proximate inflow edges thereof; and attaching the outer wrap to the inner skirt and to the outer wrap backings proximate respective outflow edges thereof.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

The foregoing and other features and advantages of the present disclosure will be apparent from the following description of embodiments hereof as illustrated in the accompanying drawings. The accompanying drawings, which are incorporated herein and form a part of the specification, further serve to explain the principles of the present disclosure and to enable a person skilled in the pertinent art to make and use the embodiments of the present disclosure. The drawings may not be to scale.
FIG. 1 depicts a transcatheter heart valve prosthesis in accordance with embodiments hereof.
FIG. 1A is an outflow or top view of the prosthesis of FIG. 1.
FIG. 1B is a perspective inflow or bottom view of the prosthesis of FIG. 1.
FIG. 2 depicts a side view of a frame segment configured for use in the prosthesis of FIG. 1.
FIG. 3 depicts a frame, having the frame segment of FIG. 2, in a flattened state for purposes of illustrating features in accordance with embodiments hereof.
FIG. 4 depicts a side view of a partially assembled transcatheter heart valve prosthesis in accordance with embodiments hereof.
FIG. 4A depicts a top view of the partially assembled transcatheter heart valve prosthesis of FIG. 4.
FIG. 4B depicts a valve leaflet, in an as-cut state, in accordance with embodiments hereof.
FIG. 4C depicts an inner skirt, in an as-cut state, in accordance with embodiment hereof.
FIG. 4D depicts a margin of attachment between a valve leaflet as depicted in FIG. 4B and an inner skirt as depicted in FIG. 4C.
FIG. 5 depicts a method of assembling a heart valve prosthesis in accordance with embodiments hereof.
FIGS. 6A and 6B depict a first operation of the method of FIG. 5 in which a valve-skirt assembly is tacked to a frame in accordance with embodiments hereof.
FIGS. 7A, 7B and 7C depict a second operation of the method of FIG. 5 in which an inner skirt of the valve-skirt assembly is attached to the frame in accordance with embodiments hereof.
FIGS. 8A and 8B depict a third operation of the method of FIG. 5 in which commissures of the valve-skirt assembly are attached to the frame in accordance with embodiments hereof.
FIG. 9 depicts an outer wrap backing, in an as-cut state, in accordance with embodiments hereof.
FIGS. 9A, 9B and 9C depict a fourth operation of the method of FIG. 5 in which the outer wrap backing of FIG. 9 is attached to the inner skirt in accordance with embodiments hereof.
FIGS. 10A and 10B depict a fifth operation of the method of FIG. 5 in which the outer wrap backing and the inner skirt are attached to the frame in accordance with embodiments hereof.
FIG. 11 depicts an outer wrap, in an as-cut state, in accordance with embodiments hereof.
FIGS. 11A and 11B depict a seventh operation of the method of FIG. 5 in which the outer wrap and the inner skirt are attached to the frame in accordance with embodiments hereof.
FIG. 12 depicts an eighth operation of the method of FIG. 5 in which the outer wrap and the inner skirt are attached at their inflow ends in accordance with embodiments hereof.
FIG. 13 depicts a ninth operation of the method of FIG. 5 in which the outer wrap and the inner skirt are attached at their outflow ends in accordance with embodiments hereof.

### DETAILED DESCRIPTION

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). The following detailed description is merely exemplary in nature and is not intended to limit the invention of the application and uses of the invention. Furthermore, there is no intention to be bound by any expressed or implied theory presented in the preceding field of the invention, background, summary or the following detailed description.

As used in this specification, the singular forms "a", "an" and "the" specifically also encompass the plural forms of the terms to which they refer, unless the content clearly dictates otherwise. The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%. It should be understood that use of the term "about" also includes the specifically recited number of value.

As used herein, the terms "above", "below", "top", "bottom", "left", and "right" are used in reference to a heart valve prosthesis when the outflow end is oriented above the inflow end. Thus, "above" is used herein to describe a direction towards the outflow end of the prosthesis, "below" is used herein to describe a direction towards the inflow end of the prosthesis, "top" is used herein to describe a position proximate to or at the outflow end of the prosthesis, "bottom" is used herein to describe a position proximate to or at the inflow end of the prosthesis, "left" is used herein to describe a direction to the left when viewing the prosthesis from the side and the outflow end is disposed above the inflow end, and "right" is used herein to describe a direction to the right when viewing the prosthesis from the side and the outflow end is disposed above the inflow end.

As used herein, the terms "radial", "radially", "radially inward" or "radially outward" describe a direction relative to a longitudinal axis of a tubular frame of the prosthesis. Thus, "radially inward" is used herein to describe a direction from a circumference toward the longitudinal axis of the tubular frame and "radially outward" is used herein to describe a direction going outward from the longitudinal axis of the tubular frame.

As used herein, the terms "proximate" or "adjacent" mean substantially next to, near, close by, and/or in proximity to. The terms "generally" and "substantially" mean approximately. When used to describe angles such as "substantially parallel" or "substantially perpendicular" the term "substantially" means within 10 degrees of the angle. When used to describe shapes such as "substantially" or "generally" cylindrical or "substantially" or "generally" tube-shaped or "generally" or "substantially" conical, the terms mean that the shape would appear cylindrical or tube-shaped or conical to a person of ordinary skill in the art viewing the shape with a naked eye. The term "about" as used herein to refer to dimensions means within 5% of the dimension.

Embodiments hereof relate to a transcatheter heart valve prosthesis and a method of assembling the transcatheter heart valve prosthesis. In particular disclosed herein are a valve component, an inner skirt, an outer wrap backing and an outer wrap that are strategically stitched/sewn to one another and/or a frame to form a transcatheter heart valve prosthesis.

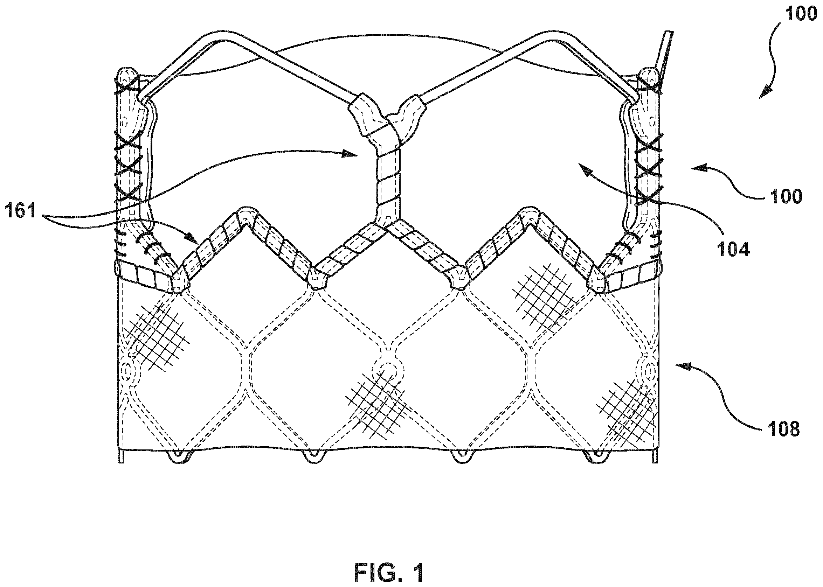

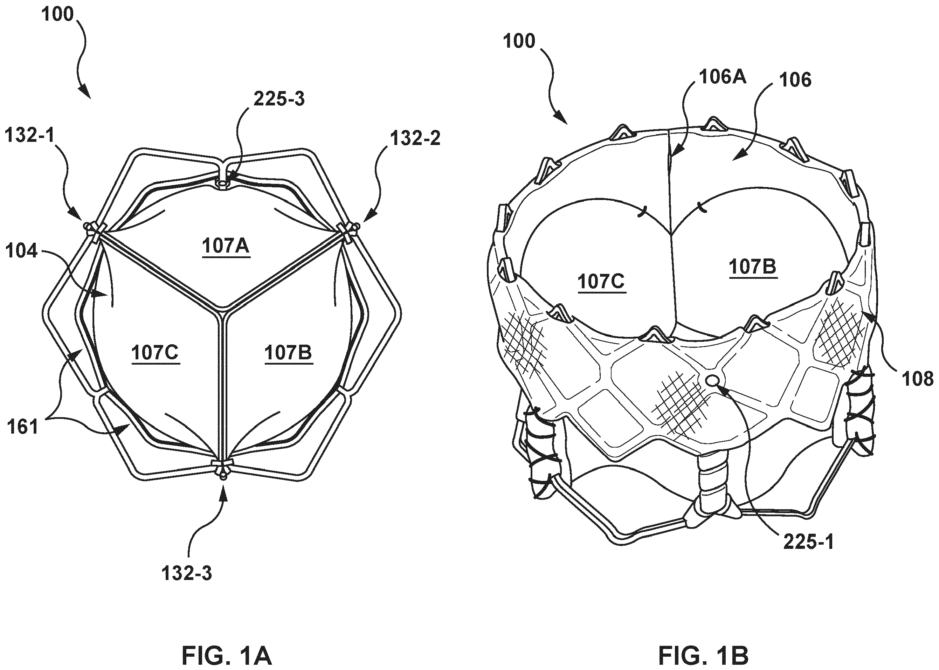

A transcatheter heart valve prosthesis 100 in accordance with embodiments hereof is shown in FIG. 1, with FIG. 1A being an outflow or top view of the prosthesis and FIG. 1B being a perspective inflow or bottom view of the prosthesis. The transcatheter heart valve prosthesis 100 may generally be described as including a radially expandable frame 102, a valve component 104, an inner skirt 106 and an outer wrap 108. The valve component 104, as best shown in FIG. 1A, includes three leaflets 107A, 107B, 107C that coapt with each other along free edges thereof and are configured to block flow in one direction to regulate blood flow through the prosthesis 100. The following description details how the valve component 104, the inner skirt 106, the outer wrap 108 and various other components are assembled and/or joined to the frame 102 to thereby form a transcatheter heart valve prosthesis 100 with valve leaflets 107A, 107B, 107C having unsupported margins of attachment, which will be described in further detail below.

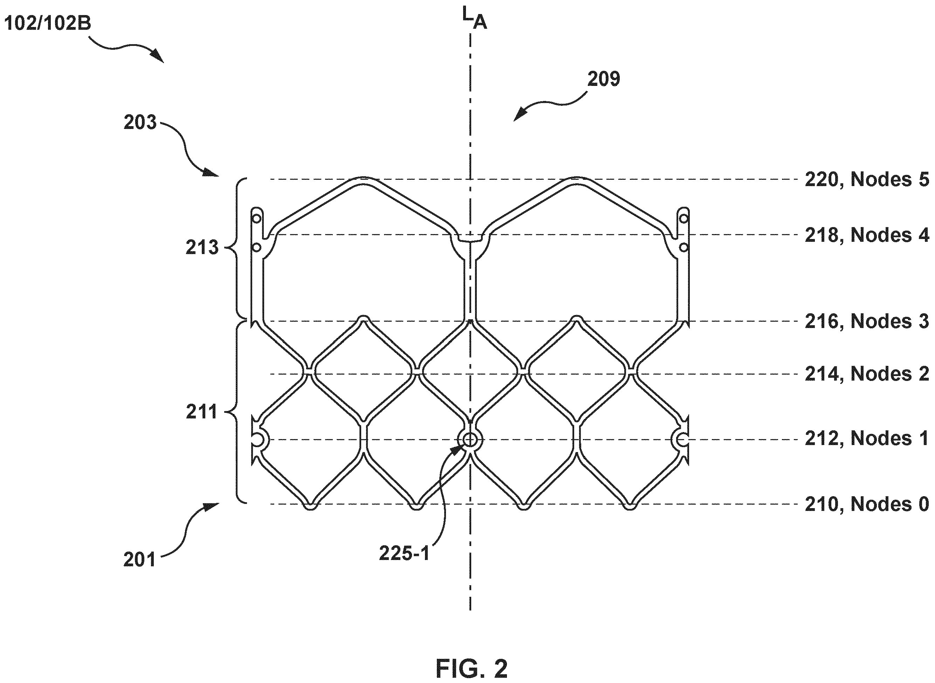

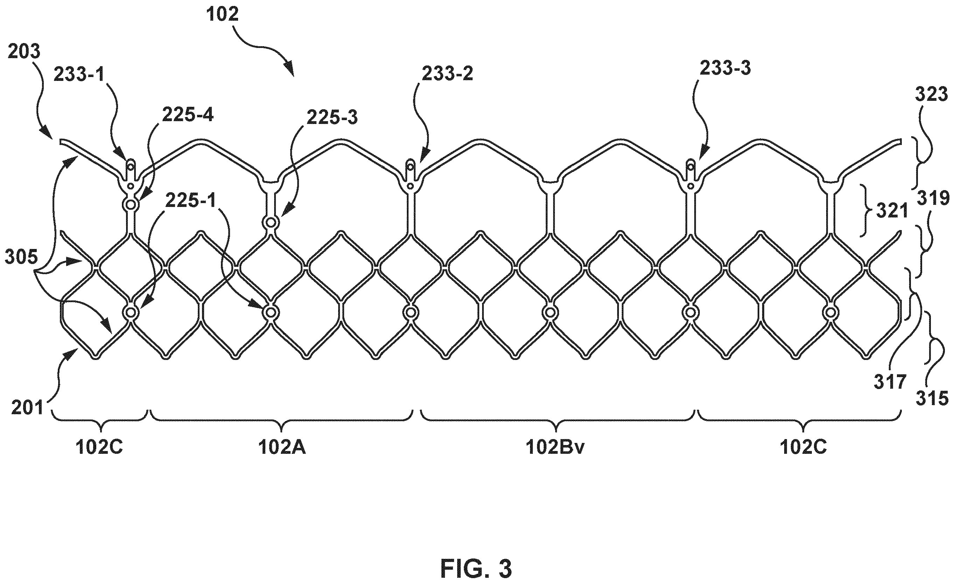

FIG. 2 depicts a side view of a second segment 102B of the frame 102 and FIG. 3 depicts first, second and third segments 102A, 102B, 102C of the frame 102 in a flattened state for purposes of illustrating features according to embodiments described herein. When assembled, a first leaflet 107A is generally disposed radially inward from the first segment 102A of the frame 102, a second leaflet 107B is generally disposed radially inward from the second segment 102B of the frame 102, and a third leaflet 107C is generally disposed radially inward from the third segment 102C of the frame 102. The frame 102 of the transcatheter heart valve prosthesis 100 has a stent structure with a plurality of struts 305 arranged to form rows of various sized cells and includes commissure posts 233-1, 233-2, 233-3 for attachment of the joined leaflets as described in detail below. The frame 102 has a generally tubular shape that defines a central lumen 209 along a longitudinal axis L_{A} thereof, as shown in FIG. 2. The central lumen 209 of the frame 102 serves as a central lumen of the transcatheter heart valve prosthesis 100. In various embodiments, a frame 102 may be configured to be balloon-expandable or self-expanding as would be understood by one of ordinary skill in the art.

In accordance with embodiments hereof a transcatheter heart valve prosthesis 100 may be configured to repair or replace a native aortic valve and an inflow section 211 of a frame 102 may be configured to anchor the prosthesis within an annulus of the native aortic valve and an outflow section 213 of the frame 102 may be configured to be disposed within a region of the aortic sinuses, proximate of the native aortic valve, while permitting blood flow therethrough to the coronary arteries. The frame 102 defines an inflow end 201 and an outflow end 203 of the transcatheter heart valve prosthesis 100, with the inflow section 211 and the outflow section 213 of the frame 102 longitudinally extending from the respective inflow and outflow ends 201, 203. The inflow section 211 is formed from first, second and third strut rows 315, 317, 319 of struts 305 and the outflow section 213 is formed from a fourth strut row 321 of longitudinally extending struts 305 and a fifth strut row 323 of struts 305 that form the outflow end 203. In an embodiment, the first strut row 315 may be a bottom row of struts 305 of the inflow section 211, the second strut row 317 may be a middle row of struts 305 of the inflow section 211, and the third strut row 319 may be a top row of struts 305 of the inflow section 211.

As shown in FIG. 2, the frame 102 also includes an inflow node row 210 of nodes 0, a first node row 212 of nodes 1, a second node row 214 of nodes 2, a third node row 216 of nodes 3, a fourth node row 218 of nodes 4, and a fifth or outflow node row 220 of nodes 5. Nodes 0 in the inflow node row 210 are crowns between the struts 305 at the inflow end 201 of the frame 102, whereas nodes 5 in the fifth node row 220 are crowns between struts 305 at the outflow end 203 of the frame 102. Nodes 1 in the first node row 212 are at junctures between struts 305 of the first and second strut rows 315, 317 and every other node 1 includes a circular marker 225-1. Nodes 2 in the second node row 214 are at junctures between struts 305 of the second and third strut rows 317, 319. Nodes 3 in the third node row 216 are crowns between two adjacent struts 305 in the third strut row 319 or are junctures of two adjacent struts 305 of the third strut row 319 and one longitudinally extending strut 305 in the fourth strut row 321. One of the nodes 3 includes a circular marker 225-3 that longitudinally aligns with a circular marker 225-1 to specify a position within the frame segment 102A of the frame 102 at which to align a center of the first leaflet 107A during assembly, as described below. Finally, nodes 4 in the fourth node row 218 are at junctures between longitudinally extending struts 305 of the fourth strut row 321 and two adjacent outflow struts 305 of the fifth strut row 323, and a circular marker 225-4 is disposed just below one of the nodes 4 to longitudinally align with a circular marker 225-1 for enabling rotational alignment of the implant to the native anatomy.

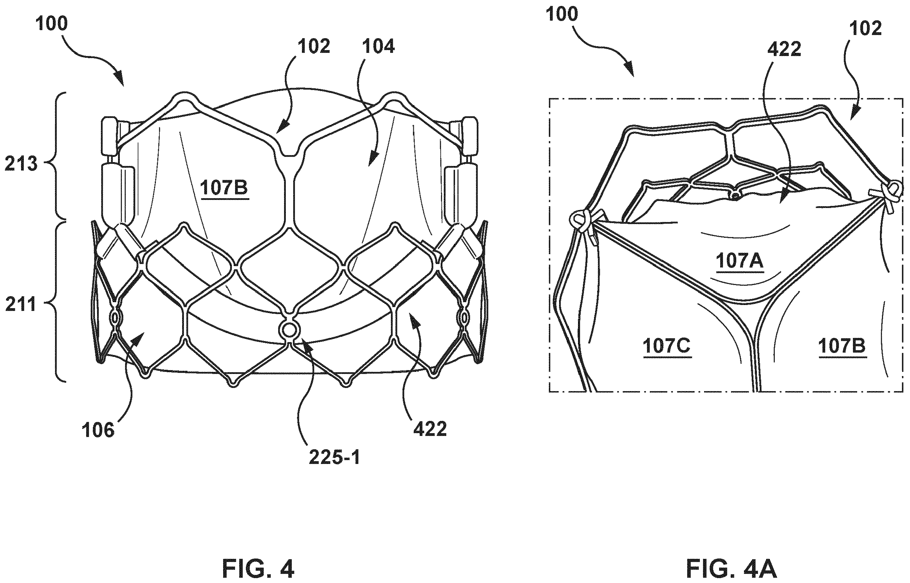

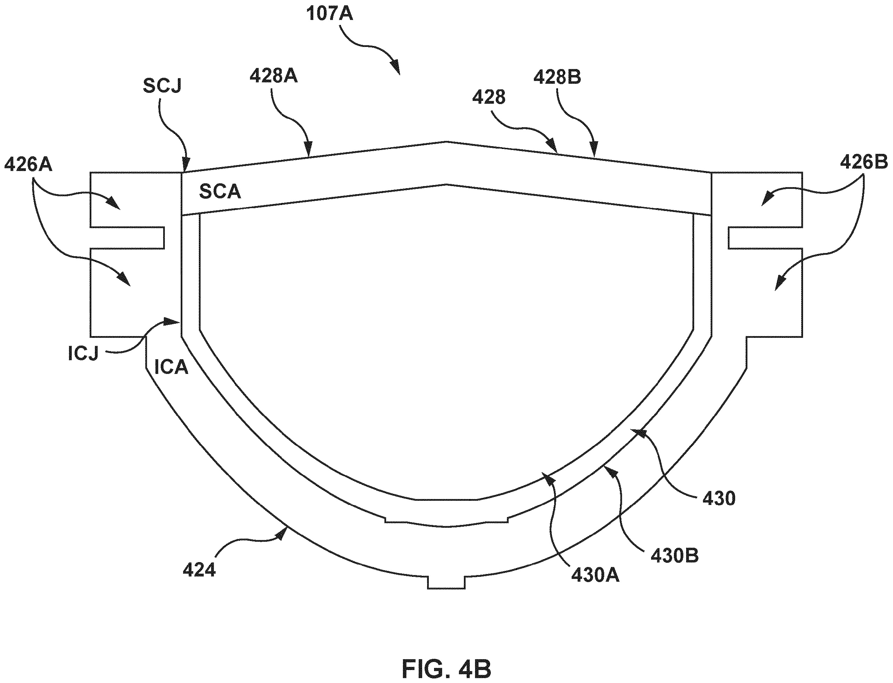

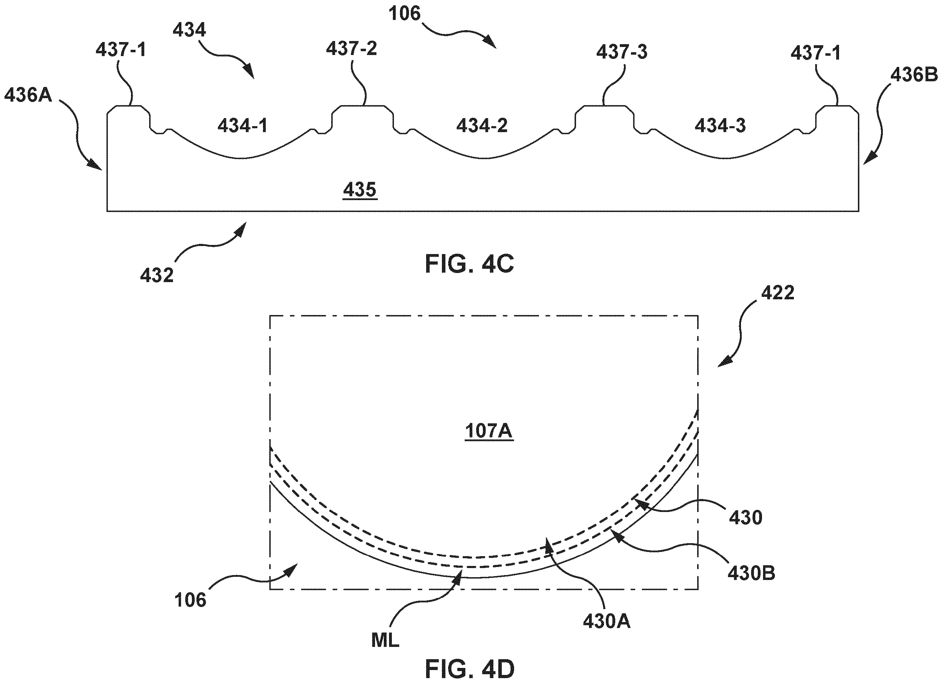

FIG. 4 is a side view of a partially assembled transcatheter heart valve prosthesis 100, with FIG. 4A being a portion of a top view of the partially assembled transcatheter heart valve prosthesis 100. The valve component 104 of the transcatheter heart valve prosthesis 100 is capable of regulating flow therethrough via valve leaflets 107A, 107B, 107C that may form a replacement valve. Generally, with reference to FIG. 4, the inner skirt 106 is attached to the valve component 104 and together these structures are disposed within an interior of the frame 102 and then joined thereto. Stated another way, the leaflets 107A, 107B, 107C are sewn to the inner skirt 106 and the resulting valve-skirt assembly 422 is disposed within the central lumen 209 of the frame 102 and sewn thereto, as will be described in detail below.

With reference to FIG. 4B, each of the leaflets 107A, 107B, 107C are intended to be substantially identical to one another, and the structures shown and described with reference to the leaflet 107A should be understood to apply to the remaining leaflets 107B, 107C. FIG. 4B shows a flat, as-cut leaflet 107A according to embodiments herein. The leaflet 107A has an outer edge 424, opposing commissure tabs 426A, 426B, and a free edge 428. The outer edge 424 of the leaflet 107A is of a substantially semi-circular shape with a respective commissure tab 426A, 426B extending from each end thereof to the free edge 428 of the leaflet 107A. The free edge 428 extends between the opposing commissure tabs 426A, 426B and includes two substantially straight segments 428A, 428B that meet at an apex at a midpoint of the free edge 428, as shown in FIG. 4B. The leaflet 107A further includes a margin of attachment 430 (described in detail below) that runs substantially parallel to the outer edge 424 of the leaflet and is spaced inward thereof.

The leaflets 107A, 107B, 107C may be formed of various flexible biocompatible materials including, but not limited to natural pericardial material such as tissue from bovine, equine or porcine origins, or synthetic materials such as polytetrafluoroethylene (PTFE), DACRON^{®} polyester, pyrolytic carbon, or other biocompatible materials. With certain prosthetic leaflet materials, it may be desirable to coat one or both sides of the replacement valve leaflet with a material that will prevent or minimize overgrowth. It is further desirable that the prosthetic leaflet material is durable and not subject to stretching, deforming, or fatigue.

Adjoining leaflets 107A, 107B, 107C are sewn to one another at their commissure tabs 426A, 426B to create commissures 132-1, 132-2, 132-3, as best shown in FIG. 1A. More particularly, the commissure 132-1 is formed by joining tabs 426A, 426B of respective leaflets 107C, 107A, the commissure 132-2 is formed by joining tabs 426A, 426B of respective leaflets 107A, 107B, and the commissure 132-3 is formed by joining tabs 426A, 426B of respective leaflets 107B, 107C. With reference to FIGS. 1A and 4B, each commissure 132-1, 132-2, 132-3 includes at its upper or outflow end a superior commissure junction SCJ, with a superior commissure area SCA defined within 5 mm thereof and includes at its lower or inflow end an inferior commissure junction ICJ, with an inferior commissure area ICA defined within 5 mm thereof.

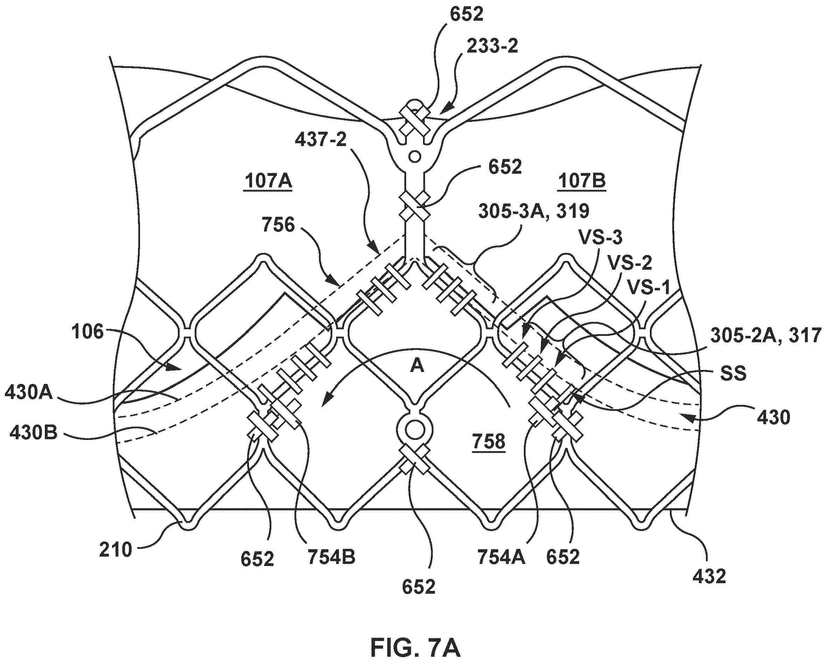

The inner skirt 106, which is shaped to enclose or line a portion of the inflow section 211 of the frame 102, will now be described with reference to FIG. 4C that shows the inner skirt 106 in a flattened state. The inner skirt 106 is formed of graft material that may be composed of woven or knitted fabric, pericardial tissue, or a polymer material, and is configured to be situated between the leaflets 107A, 107B, 107C and an interior surface of the frame 102, as discussed below. The inner skirt 106 includes a straight inflow end or edge 432, a scalloped outflow end or edge 434, a first side edge 436A, a second side edge 436B, an interior surface 435, and an exterior surface 758 (as first discussed with reference to FIG. 7A). The first side edge 436A and the second side edge 436B extend between the inflow edge 432 and the outflow edge 434, as shown in FIG. 4C, and are sewn to each other to form the tubular structure of the inner skirt 106. The inner skirt 106 includes cut-outs 434-1, 434-2, 434-3 equally spaced along the scalloped outflow edge 434, with each cut-out 434-1, 434-2, 434-3 having a substantially semi-circular shape. As also shown in FIG. 4C, the inner skirt 106 includes peak regions 437-1, 437-2, 437-3 along the outflow edge 434, where a height of the inner skirt 106 is greatest, that are positioned between respective cut-outs 434-1, 434-2, 434-3. In accordance with embodiments disclosed herein, the cut-outs 434-1, 434-2, 434-3 of the inner skirt 106 contribute to a transcatheter heart valve prosthesis 100 having an overall reduced profile and thereby allows for lower insertion forces during the crimping process thereof. Each of the cut-outs 434-1, 434-2, 434-3 receives a respective leaflet 107A, 107B, 107C therein, as described below.

As noted above, the inner skirt 106 when assembled with the valve component 104, *i.e.,* with the three leaflets 107A, 107B, 107C, is referred to as the valve-skirt assembly 422. To form the valve-skirt assembly 422, an outer edge 424 of a leaflet 107A, 107B, 107C is positioned to overlap the interior surface 435 of the inner skirt 106 at a respective cut-out 434-1, 434-2, 434-3 of the outflow edge 434. Each of the leaflets 107A, 107B, 107C is then sewn to the inner skirt 106 via sutures at an inner line of stitches 430A and an outer line of stitches 430B that run parallel to each other and to the outer edge 424 of the leaflet, as best shown in FIGS. 4B and 4D. The margin of attachment 430 is defined as the area between the inner line of stitches 430A and the outer line of stitches 430B. Once each of the leaflets 107A, 107B, 107C is sewn to the inner skirt 106 by its respective inner and outer lines of stitches 430A, 430B, the leaflets 107A, 107B, 107C are sewn to one another at their commissure tabs 426A, 426B so as to form commissures 132-1, 132-2, 132-3 as described above. As best shown in FIG. 1B, the first side edge 436A and the second side edge 436B of the inner skirt 106 are joined to create a side seam 106A that is aligned with the commissure 132-3 formed between and including the leaflets 107B, 107C. The side seam 106A and the commissure 132-3 thereby secure the valve-skirt assembly 422 in a tubular configuration. The valve-skirt assembly 422 so formed is ready for attachment to the frame 102 of the transcatheter heart valve prosthesis 100, as will be described in detail below.

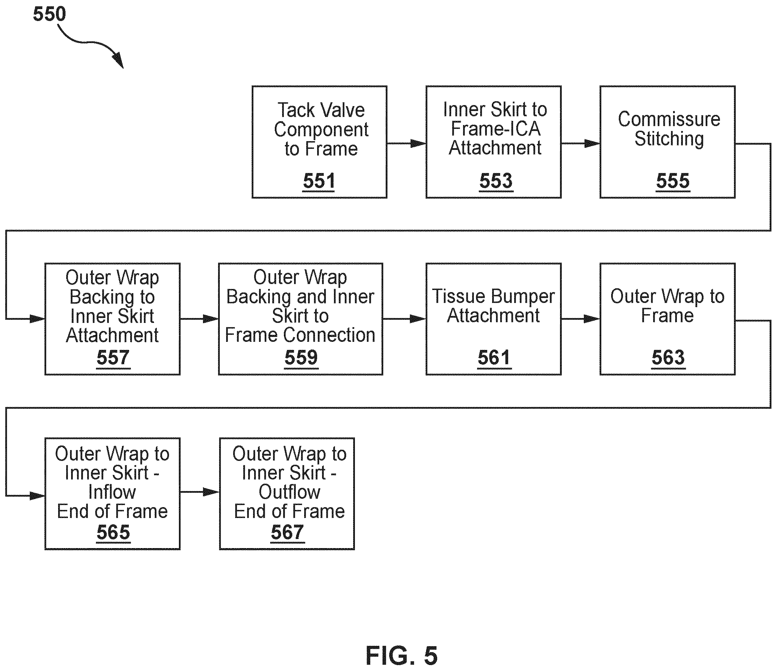

A method of assembling a transcatheter heart valve prosthesis 100 will now be described. FIG. 5 is a block diagram of a method 550 of assembling a transcatheter heart valve prosthesis 100, which includes attaching a valve-skirt assembly 422, an outer wrap 108, and various other components to a frame 102.

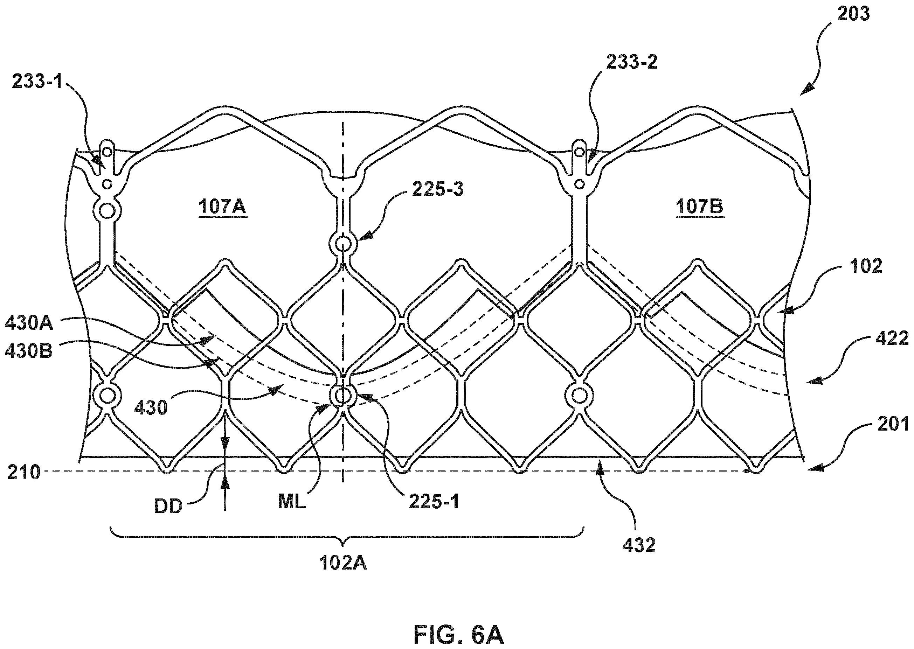

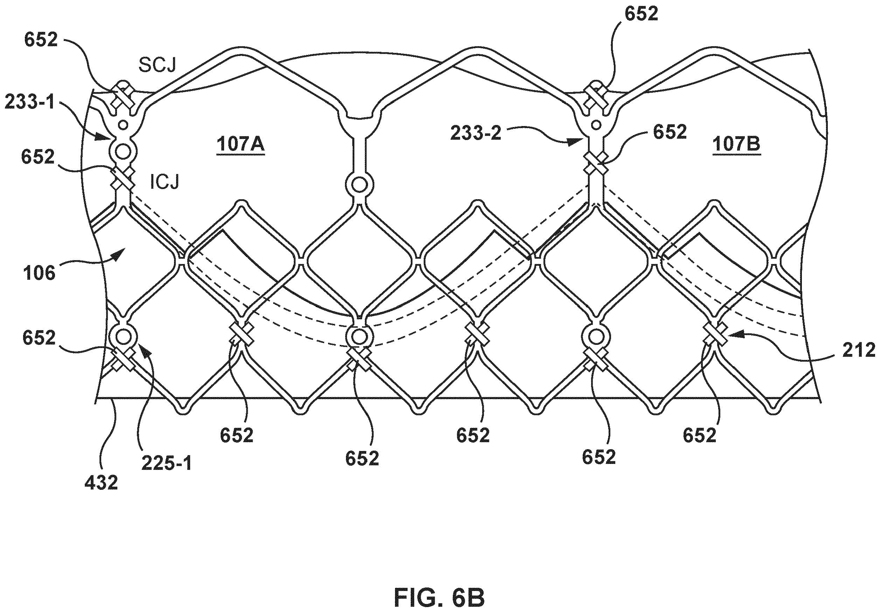

In a first operation 551 of the method 550, the valve-skirt assembly 422 is tacked to the frame 102, as illustrated by FIGS. 6A and 6B. Initially, the valve-skirt assembly 422 (only a portion of which is shown in FIG. 6A for clarity) is disposed within the central lumen 209 of the frame 102 so that the first leaflet 107A is positioned in the frame segment 102A with the inflow edge 432 of the inner skirt 106 aligned in parallel with the inflow end 201 of the frame 102. The inflow edge 432 of the inner skirt 106 is spaced from the inflow crowns or nodes 0 of the inflow node row 210 of the frame 102, so as to be spaced a defined distance DD from the inflow crowns to thereby leave the inflow crowns exposed. A midline ML of the first leaflet 107A is centered in the frame segment 102A opposite of the aligned circular markers 225-1, 225-3 such that its margin of attachment 430 is aligned with the circular marker 225-1, whereby the inner line of stitches 430A is positioned above the circular marker 225-1 and the outer line of stitches 430B is positioned below the circular marker 225-1.

Next, a plurality of tack or temporary stitches 652 are made to hold the valve-skirt assembly 422, aligned as noted in the prior paragraph, to the frame 102. In an embodiment, a tack stitch 652 may be at each superior commissure junction SCJ and inferior commissure junction ICJ of each commissure 132-1, 132-2, 132-3 (not shown) to tack the valve tissue to the commissure posts 233-1, 233-2, 233-3 of the frame 102. In addition, a tack stitch 652 may be made at each node 1 in the first node row 212 to tack the inner skirt 106 and any excess valve tissue to the frame 102, taking care to apply tack stitches 652 below each circular marker 225-1 in the first node row 212, as best shown in FIG. 6B.

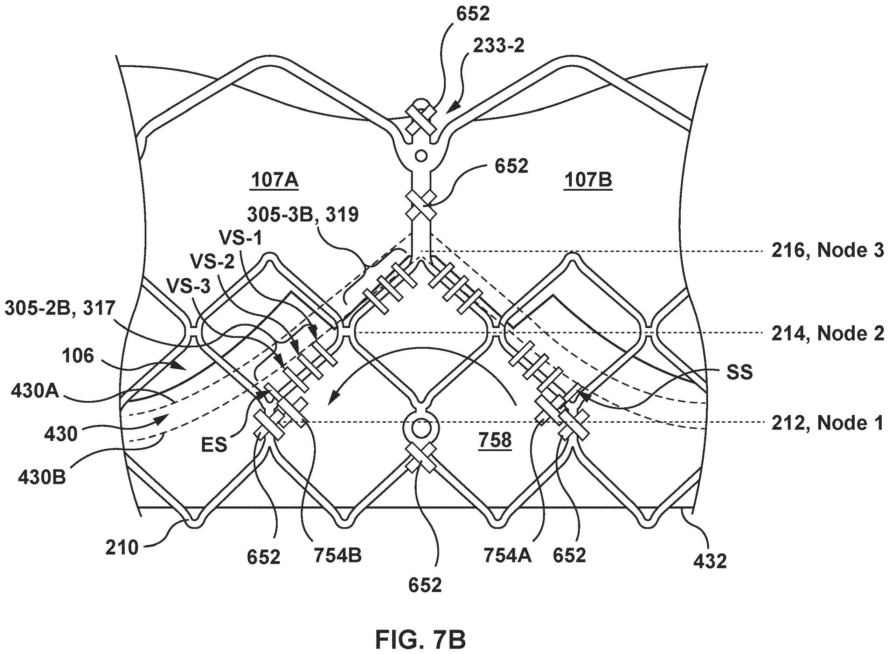

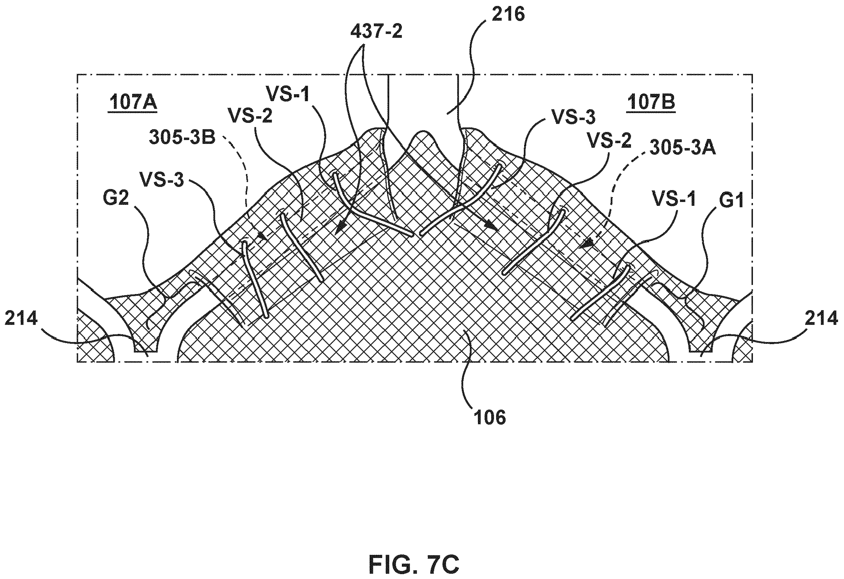

In a second operation 553 of the method 550, the inner skirt 106 of the valve-skirt assembly 422 is attached to the frame 102 in the inferior commissure area ICA below each commissure 132-1, 132-2, 132-3 (not shown), as illustrated in FIGS. 7A, 7B and 7C that show the inner skirt 106 within the frame 102, or stated another way show the frame 102 exterior to and surrounding the inner skirt 106. After performing the first operation 551, and as illustrated in FIG. 7A, a peak region 437-2 of the inner skirt 106 is aligned with the commissure post 233-2 of the frame 102, and although not shown the remaining peak regions 437-1, 437-3 are also aligned with their respective commissure posts 233-1, 233-3. In the second operation 553, a pattern of stitches 756 to join the valve-skirt assembly 422 to the frame 102 in the inferior commissure area ICA is made proceeding in a counter-clockwise direction from a starting knot 754A to an end knot 754B, as represented by arrow A in FIG. 7A.

Initially a starting knot 754A is made proximate a tack stitch 652 at a node 1, which is located below and to a first or right side of the commissure post 233-2. The starting knot 754A is created by inserting a needle (not shown), carrying a suture having a looped end (not shown), through an exterior surface 758 of the inner skirt 106 at a point just below a strut 305-2A of the second strut row 317 and pulling the needle through in a direction from the outside to the inside of the frame 102 until the looped end of the suture contacts the exterior surface 758 of the inner skirt 106. The needle is then inserted through the valve tissue and the interior surface 435 of the inner skirt 106 at a point just above the outer line of stitches 430B within the margin of attachment 430 and pulled through in a direction from the inside to the outside of the frame 102. Thereafter, the needle is inserted through the looped end of the suture thereby creating a starting stitch SS wrapped across the strut 305-2A and a flat knot is then made to secure in place the starting knot 754A.

The pattern of stitches 756 continues by making a set of three vertical stitches VS-1, VS-2, VS-3 around the strut 305-2A of the second strut row 317. The set of three vertical stitches VS-1, VS-2, VS-3 are located along the strut 305 between the starting stitch SS near the node 1 and the node 2. The first vertical stitch VS-1 is created around the strut 305 by inserting and interiorly pulling the needle and suture through the initial suture hole in the exterior surface 758 of the inner skirt 106 (below the strut), inserting and exteriorly pulling the needle and suture back through the valve tissue and the inner skirt 106 at a point just above the outer line of stitches 430B within the margin of attachment 430, and inserting and interiorly pulling the needle and suture through a point just below the strut 305-2A, from the exterior surface 758 to the interior surface 435 of the inner skirt 106, to thereby wrap the suture around the outer surface of the strut 305-2A and create the first vertical stitch VS-1. Second and third vertical stitches VS-2, VS-3 are created by repeating these steps such that the vertical stitches VS-1, VS-2, VS-3 and starting stitch SS pass through the valve tissue and the inner skirt 106 at respective points just above the outer line of stitches 430B within the margin of attachment 430 of the second leaflet 107B, and through the inner skirt 106 below the strut 305-2A so as to be evenly spaced between nodes 1 and 2 along the strut 305-2A, as shown in FIG. 7A. Accordingly, the margins of attachment 430 of the three leaflets 107A, 107B, 107C are sewn to the frame 102 at the inferior commissure areas ICA but are not sewn to the frame 102 at a bottom portion of the margin of attachments 430, as best shown in FIG. 4. Thus, the margins of attachment 430 of the three leaflets 107A, 107B, 107C, at the bottom of each leaflet, are unsupported by the frame 102 to prevent leaflet tearing.

In the second operation 553, with reference to FIGS. 7A-7C, three vertical stitches VS-1, VS-2, VS-3 are sewn in a similar manner as just described around a strut 305-3A in the third strut row 319 of the frame 102 and then around a strut 305-3B in the third strut row 319 of the frame 102. Colloquially, the strut 305-3A may be described as extending between a node 2 and a node 3 on the first or right side of the commissure post 233-2, and the strut 305-3B may be described as extending between the node 3 and a node 2 on a second or left side of the commissure post 233-2.

When making the vertical stitches VS-1, VS-2, VS-3, excess graft material or fabric from the peak region 437-2 of the inner skirt 106 is wrapped over and around the respective struts 305-3A, 305-3B from the inside to the outside of the frame 102, such that the graft material or fabric folds over an exterior surface of the struts 305-3A, 305-3B. Of note, a gap G1, G2 of an exposed or uncovered segment of the strut 305-3A, 305-3B is maintained proximate its respective node 2 in the second node row 214, as shown in FIG. 7C. In an embodiment, the gap G1, G2 of exposed strut 305-3A, 305-3B may be approximately 2 mm wide to accommodate the later placement of a tissue bumper as discussed below.

When making the vertical stitches VS-1, VS-2, VS-3 around the strut 305-3A, each of the vertical stitches VS-1, VS-2, VS-3 is made to pass through tissue of the second leaflet 107B, at respective points just above the outer line of stitches 430B within the margin of attachment 430, along with passing through the folded or wrapped graft material of the inner skirt 106 as noted above. Similarly, when making the vertical stitches VS-1, VS-2, VS-3 around the strut 305-3B, each of the vertical stitches VS-1, VS-2, VS-3 is made to pass through tissue of the first leaflet 107A, at respective points just above the outer line of stitches 430B within the margin of attachment 430, along with passing through the folded or wrapped graft material of the inner skirt 106 as noted above.

In the second operation 553, the pattern of stitches 756 is completed by making a set of three vertical stitches VS-1, VS-2, VS-3 and an end stitch ES around the strut 305-2B of the second strut row 317 as shown in FIG. 7B. The three vertical stitches VS-1, VS-2, VS-3 and the end stitch ES that wrap around the strut 305-2B pass through tissue of the first leaflet 107A, at respective points just above the outer line of stitches 430B within the margin of attachment 430, along with passing through the inner skirt 106 as similarly described for the three vertical stitches VS-1, VS-2, VS-3 and starting stitch SS of the strut 305-2A. Finally, with the same suture, the end knot 754B may be tied proximate the tack stitch 652 at node 1 in the first node row 212.

The pattern of stitches 756 is then repeated to sew the valve tissue and the remaining peak regions 437-1, 437-3 of the inner skirt 106 of the valve-skirt assembly 422 to the frame 102 in the inferior commissure area ICA below the remaining commissure posts 233-1, 233-3. Once the second operation 553 is complete, the tack stitches 652 located at nodes 1 beneath either a starting or end knot 754A, 754B are removed.

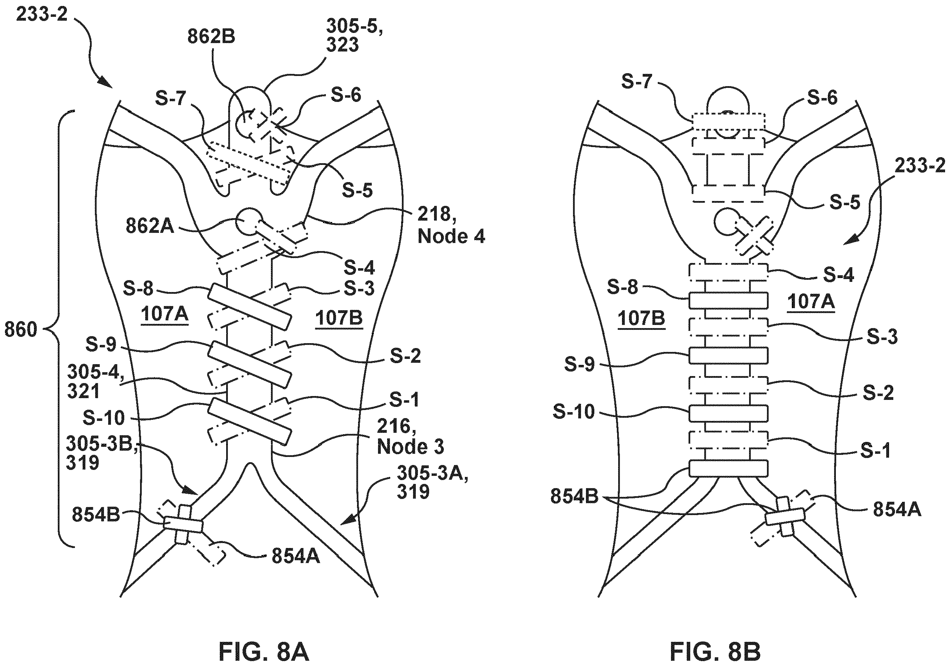

As stated previously, adjacent leaflets 107A, 107B, 107C are attached to one another at their lateral commissure tabs 426A, 426B to form commissures 132-1, 132-2, 132-3 therebetween. In a third operation 555 of the method 550, each commissure 132-1, 132-2, 132-3 is attached to a respective commissure post 233-1, 233-2, 233-3 of the frame 102. The third operation 555 will be described with reference to FIG. 8A that depicts an exterior view of a commissure attachment pattern 860 for attaching a commissure (not shown) to a commissure post 233-2 and FIG. 8B that depicts an interior view of the commissure attachment pattern 860 with the commissure removed.

With reference to FIGS. 3 and 8A, each commissure post 233-1, 233-2, 233-3 is comprised of a longitudinally extending strut 305-4, which is located in the fourth strut row 321 and will be referred to alternatively as a commissure bar 305-4, and an outflow strut 305-5 aligned therewith, which is located in the fifth strut row 323 and will be referred to alternatively as a commissure tab 305-5. Each commissure tab 305-5 of the commissure posts 233-1, 233-2, 233-3 includes a lower hole 862A and an upper hole 862B therethrough, as shown in the detail of FIGS. 8A and 8B.

In the third operation 555, a needle with a suture are used to create the commissure attachment pattern 860. To begin a starting knot/stitch 854A is made over the strut 305-3B, which when viewed from the exterior as shown in FIG. 8A is located below and to the left of the node 3 at a base of the commissure post 233-2. Next, three stitches S-1, S-2, S-3 are created across the commissure bar 305-4 and a fourth stitch S-4 is formed as a locking stitch at the node 4 that passes through the lower hole 862A of the commissure tab 305-5. A fifth stitch S-5 is made on the commissure tab 305-5 to be located between the lower and upper holes 862A, 862B, and a sixth stitch S-6 is formed as a locking stitch that passes through the upper hole 862B of the commissure tab 305-5. Following the same path but reversed, a seventh stitch S-7 is made to create an X-stitch with the fifth stitch S-5, an eight stitch S-8 is made to create an X-stitch with the third stitch S-3, a ninth stitch S-9 is made to create an X-stitch with the second stitch S-2, and a tenth stitch S-10 is made to create an X-stitch with the first stitch S-1. An end knot 854B is then made at the same location as the starting knot/stitch 854A to complete the commissure attachment pattern 860. Those skilled in the art will understand that the total number of X-stitches can vary depending on the overall size of the transcatheter heart valve prosthesis 100. With reference to FIG. 8B, it should be understood by one of skill in the art that when creating the interiorly placed horizontal portions of the first through tenth stitches S-1 through S-10 the needle and suture are passed through tissue of the commissure (not shown) to secure the commissure to the commissure post, for instance, to secure the commissure 132-2 to the commissure post 233-2 shown in FIGS. 8A and 8B. In an embodiment, a topmost stitch of each commissure attachment, for example stitches S-6 and S-7 in FIGS. 8A and 8B, may be at least 0.5 mm away from a topmost edge of the tissue.

In the embodiment shown in FIG. 8A, the commissure tab 305-5 of the commissure post 233-2 receives a single X-stitch, but in other embodiments a commissure tab may receive two or more X-stitches depending on the overall size of the transcatheter heart valve prosthesis.

The commissure attachment pattern 860 is then repeated to sew the remaining commissures 132-1, 132-3 of the valve component 104 to the remaining commissure posts 233-1, 233-3 of the frame 102. During the creation of the commissure attachment patterns 860, once a stitch is made proximate to a tack stitch 652 (not shown in FIGS. 8A and 8B), the tack stitch is removed.

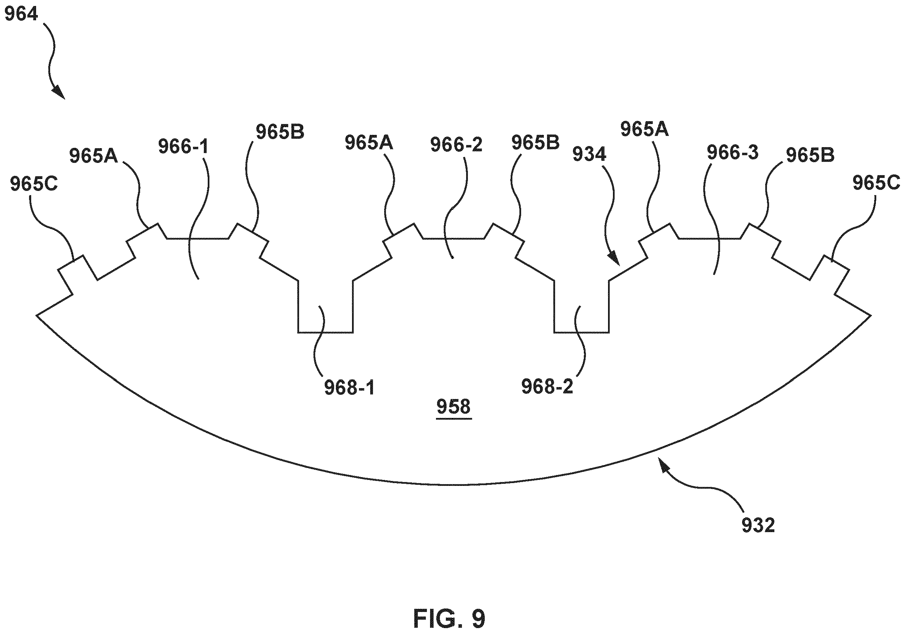

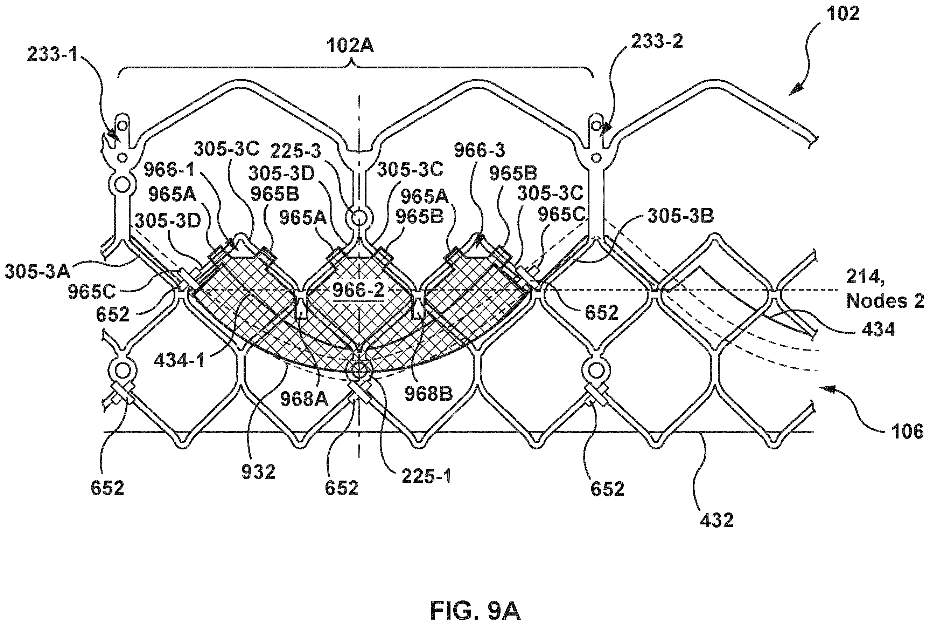

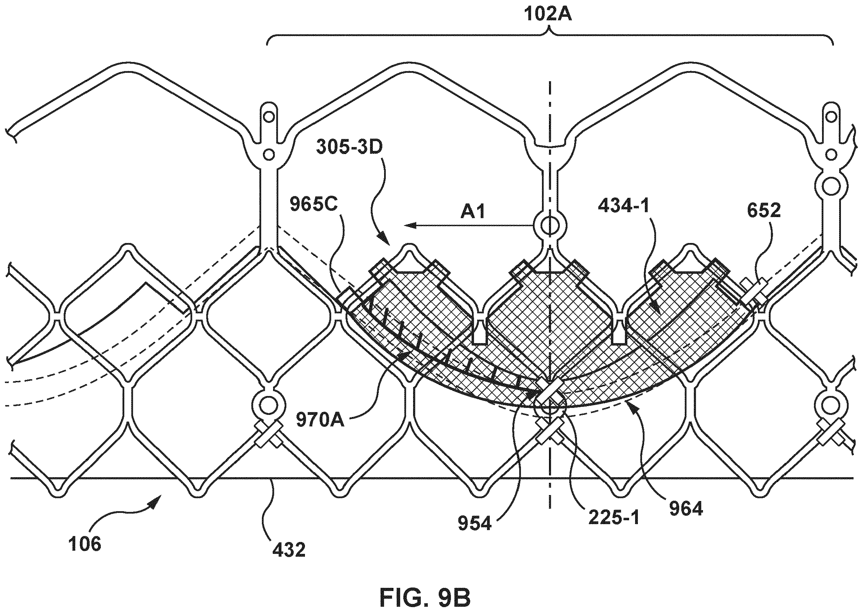

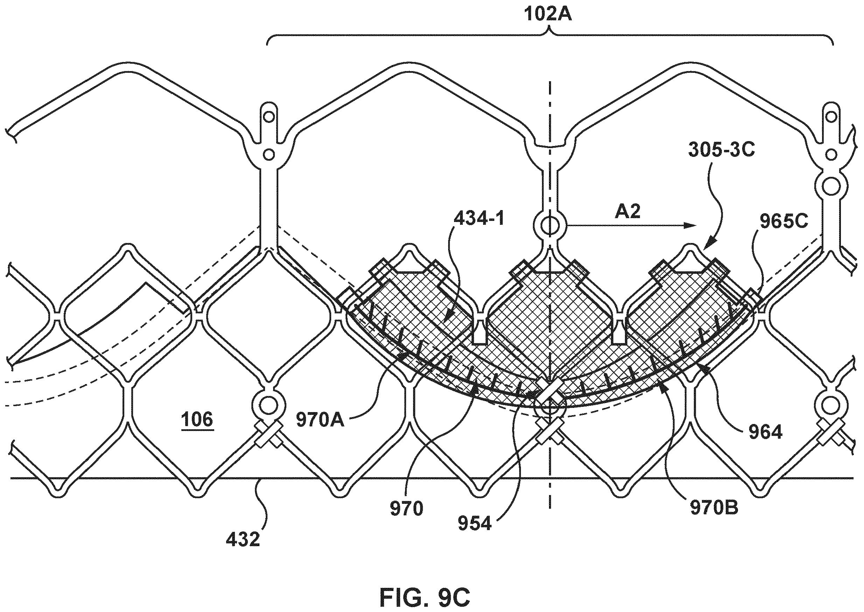

In a fourth operation 557 of the method 550, with reference to FIGS. 9-9C, an outer wrap backing 964 is sewn to the inner skirt 106 at each of the cut-outs 434-1, 434-2, 434-3 along the scalloped outflow edge 434 of the inner skirt 106. FIG. 9 shows a flat, as-cut outer wrap backing 964 that is configured to be situated on an exterior surface of the frame 102. The outer wrap backing 964 includes a curved inflow edge or end 932, an outflow edge or end 934, an interior surface (not shown), and an exterior surface 958. The curved inflow edge 932 of the outer wrap backing 964 has an arc shape that is sized to correspond to a respective cut-out 434-1, 434-2, 434-3 of the inner skirt 106. The outflow edge 934 of the outer wrap backing 964 includes first, second and third outflow sections 966-1, 966-2, 966-3 that are separated by first and second windows 968-1. 968-2. Each of the first, second and third outflow sections 966-1, 966-2, 966-3 includes first and second tabs 965A, 965B, and each of the first and third outflow sections 966-1, 966-3 includes a respective end tab 965C. Each of the tabs 965A, 965B, 965C is a substantially rectangular piece of the graft material or fabric of the outer wrap backing 964 that outwardly extends from its respective outflow section 966-1, 966-2, 966-3. The tabs 965A, 965B, 965C are configured to wrap around struts 305 of the frame 102 to aid in attaching the outer wrap backing 964 to the frame 102, as discussed in further detail below. In accordance with embodiments hereof, the outer wrap backing 964 is a material selected to prevent or prohibit paravalvular leakage through the valve prosthesis and may be a laser cut piece of material that is composed of a woven or knitted fabric, pericardial tissue, or a polymer material that prevents or prohibits paravalvular leakage.

FIGS. 9A-9C illustrate, in accordance with the fourth operation 557, attachment of the outer wrap backing 964 to the inner skirt 106 within the frame segment 102A. First, the outer wrap backing 964 is placed on an exterior of the frame segment 102A such that a center of the outer wrap backing 964 is longitudinally aligned with the markers 225-1, 225-3 of the frame 102 and a center of the inflow edge 932 of the outer wrap backing 964 is positioned over the marker 225-1. In addition, each of the outflow sections 966-1, 966-2, 966-3 is positioned at or just under pairs of struts 305-3C, 305-3D of the third strut row 319, as shown in FIG. 9A, so that the tabs 965A of the outer wrap backing 964 overlap with the struts 305-3D, the tabs 965B of the outer wrap backing 964 overlap with the struts 305-3C, and the end tabs 965C of the outer wrap backing 964 overlap with one of the struts 305-3C, 305-3D proximate a respective node 2. When properly positioned, the windows 968-1, 968-2 of the outer wrap backing 964 are aligned over and expose respective nodes 2 in the second node row 214. As shown in FIG. 9A, a portion of the inflow end 932 of the outer wrap backing 964 overlaps with a portion of the cut-out 434-1 in the outflow edge 434 of the inner skirt 106. To hold the outer wrap backing 964 in place, attachment tack stitches 652 are made to attach the end tabs 965C of the outer wrap backing 964 around the respective struts 305-3C, 305-3D proximate the respective nodes 2.

With reference to FIGS. 9B and 9C, a blanket-stitching pattern 970 is made to attach the inflow edge 932 of the outer wrap backing 964 to the overlapped portion of the inner skirt 106, with a portion of the frame 102 therebetween. The blanket-stitching pattern 970 is sewn with a single suture that is used to create first and second blanket stitch segments 970A, 970B that when complete run in parallel to an entire length of the cut-out 434-1 in the outflow edge 434 of the inner skirt 106. To begin the blanket-stitching pattern 970, a knot 954 is made by a suture above the marker 225-1 and the suture is divided into equal first and second segments. Working in the direction of arrow A1, the first segment of the suture is used to sew a first line of blanket stitches that extends from the marker 225-1 to the end tab 965C on the strut 305-3D, and a returning second line of blanket stitches that extends from the end tab 965C on the strut 305-3D to the marker 225-1 to thereby create the first blanket stitch segment 970A. Next working in the direction of arrow A2, the second segment of the suture is used to sew a first line of blanket stitches that extends from the marker 225-1 to the end tab 965C on the strut 305-3C, and a returning second line of blanket stitches that extends from the end tab 965C on the strut 305-3C to the marker 225-1 to thereby create the second blanket stitch segment 970B. A further knot is made at the same location as the knot 954 to secure the suture.

The first blanket stitch segment 970A attaches the outer wrap backing 964 only to the inner skirt 106, and not to the frame 102, except for securing the end tab 965C of the outer wrap backing 964 around the strut 305-3D. Similarly, the second blanket stitch segment 970B attaches the outer wrap backing 964 only to the inner skirt 106, and not to the frame 102, except for securing the end tab 965C of the outer wrap backing 964 around the strut 305-3C. To attach the end tabs 965C to their respective struts 305-3C, 305-3D, the tack stitch 652 disposed at the end tab may be removed once the first line of blanket stitches reaches the respective end tab.

To complete the fourth operation 557 of the method 550, the blanket-stitching pattern 970 is then repeated to sew an outer back wrapping 964 to the inner skirt 106 at the remaining cut-outs 434-2, 434-3 thereof.

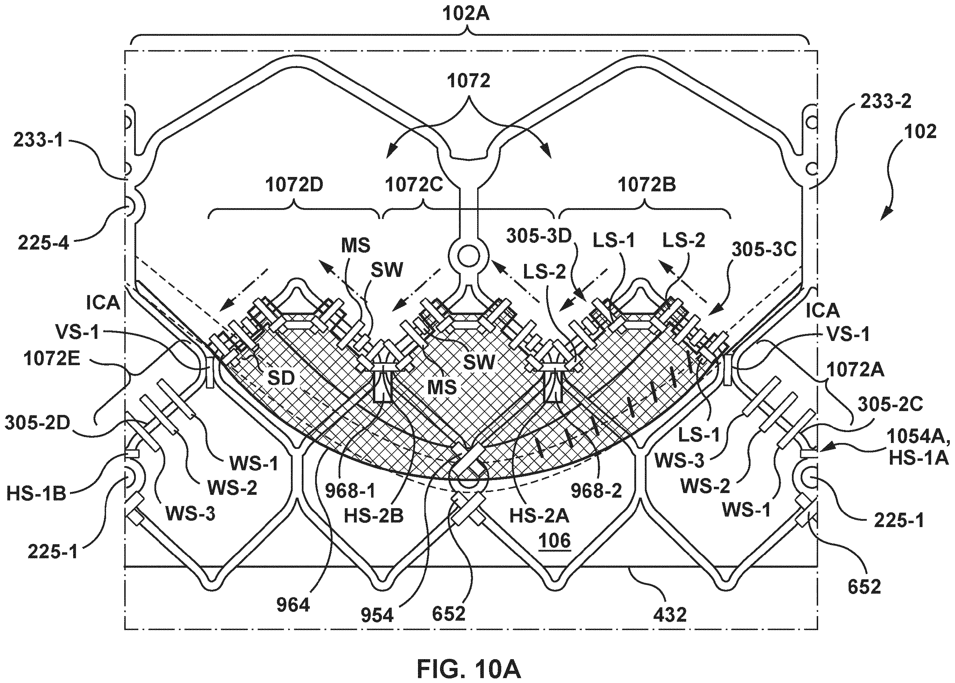

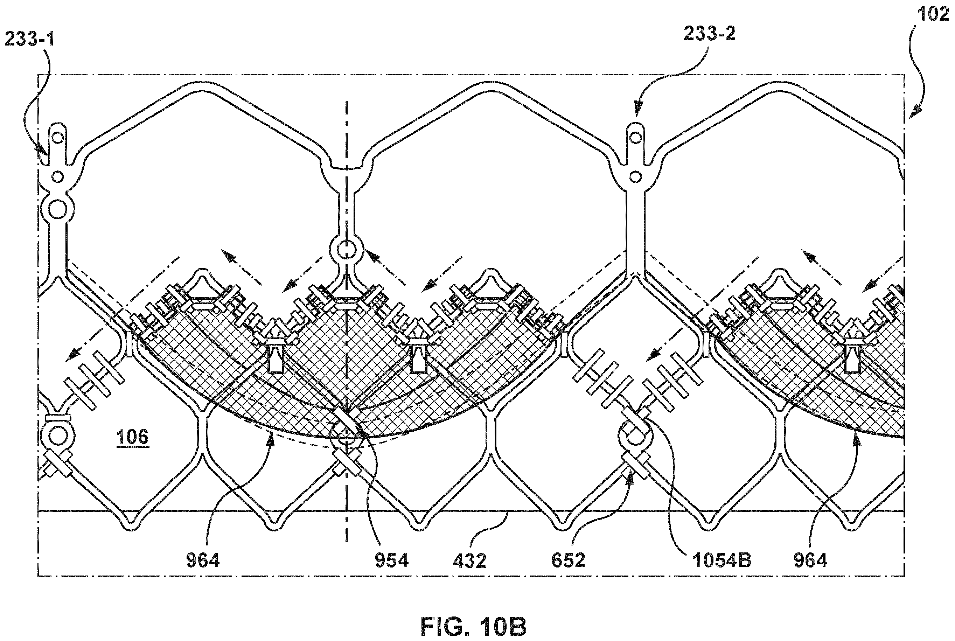

In a fifth operation 559 of the method 550, the outer wrap backings 964 and the inner skirt 106 are sewn to the frame 102 of the transcatheter heart valve prosthesis 200 as illustrated in FIGS. 10A and 10B. A backing-skirt-frame attachment pattern 1072 is utilized for this purpose that includes stitch segments 1072A, 1072B, 1072C, 1072D, 1072E as described herein. With reference to FIG. 10A, a starting knot 1054A is made with a suture in the inferior commissure area ICA of the second commissure post 233-2 to begin the backing-skirt-frame attachment pattern 1072, and particularly the starting knot 1054A is made above the marker 225-1 in the first node row 212 that longitudinally aligns with the second commissure post 223-2. A first stitch segment 1072A is then sewn with the suture to attach the inner skirt 106 to the strut 305-2C of the second strut row 317. The first stitch segment 1072A starts with a horizontal stitch HS-1A that is made essentially over the starting knot 1054A above the marker 225-1 in the first node row 212, and is continued by a plurality of whip stitches WS-1, WS-2, WS-3 that are formed to attach the inner skirt 106 along a length of the strut 305-2C, and is completed by a vertical stitch VS-1 that is made at a node 2 of the second node row 214, as shown in FIG. 10A.

The second, third and fourth stitch segments 1072B, 1072C, 1072D are then sewn with the suture in sequence, proceeding in the direction of the arrows shown in FIG. 10A, to attach the tabs 965A, 965B, 965C of the outer wrap backing 964 and the inner skirt 106 to the corresponding pairs of struts 305-3C, 305-3D, which are disposed along the top strut row 319 of the inflow section 211 between the first and second commissure posts 233-1, 233-2. The second, third and fourth stitch segments 1072B, 1072C, 1072D include locking stitches LS-1, LS-2 as the first and last stitch along each strut 305-3C, 305-3D. In addition between the locking stitches LS-1, LS-2 along each strut 305-3C, 305-3D, each of the second, third and fourth stitch segments 1072B, 1072C, 1072D includes a middle stitch MS that attaches fabric of the outer wrap backing 964 and/or the inner skirt 106 to the respective strut 305-3C, 305-3D, and a suture wrap SW where the suture is wrapped around the respective strut 305-3C, 305-3D without attaching to any fabric. In an embodiment, along each strut 305-3C, 305-3D a middle stitch MS should be spaced a stitch distance SD, of approximately 1.5 mm, from a locking stitch LS-1, LS-2 of the strut 305-3C, 305-3D that is adjacent a node 2 of the second node row 214.

A horizonal stitch HS-2A is made with the suture between the second and third stitch segments 1072B, 1072C to span the window 968-2 of the outer wrap backing 964, and a horizonal stitch HS-2B is made with the suture between the third and fourth stitch segments 1072C, 1072D to span the window 968-1 of the outer wrap backing 964, and thereby attach the outer wrap backing 964 to a respective node 2 of the frame 102.

The fifth stitch segment 1072E is made with the suture and continues from the fourth stitch segment 1072D. The fifth stitch segment 1072E includes a vertical stitch VS-1 that is made at a node 2 of the second node row 214, and is continued by a plurality of whip stitches WS-1, WS-2, WS-3 that are formed to attach the inner skirt 106 along a length of the strut 305-2D, and is completed by a horizontal stitch HS-1B that is made above the marker 225-1 that is aligned with the marker 225-4 on the first commissure post 233-1. Like the positioning of the first stitch segment 1072A, the fifth stitch segment 1072E is disposed within the inferior commissure area ICA of the first commissure post 233-1.

To complete the fifth operation 559 of the method 550, the backing-skirt-frame attachment pattern 1072 is continued around the frame 102 to sew the remaining outer back wrappings 964, and inner skirt 106 attached thereto, to the remaining frame segments 102B, 102C to thereby end the attachment pattern 1072 at an end knot 1054B, which is made proximate the start knot 1054A, as shown in FIG. 10B.

In a sixth operation 561 of the method 550, tissue bumpers 161 (shown in FIGS. 1 and 1A) are attached to cover exposed areas of the frame segments 102A, 102B, 102C that are located within the outflow section 213 of the frame 102. The tissue bumpers 161 function to prevent damage from being caused by any contact that may occur between the leaflets 107A, 107B, 107C and the frame 102 during systole. In particular, the tissue bumpers 161 are created to cover the longitudinally extending struts 305-4 of the frame 102 that are not commissure bars, or stated another way the tissue bumpers 161 are attached to the remaining longitudinally extending struts 305-4 within the outflow section 213 of the frame 102 that do not have a commissure of the valve component attached thereto. In addition, the tissue bumpers 161 are created to cover the struts 305-3 in the third strut row 319 of the frame 102, as well as the sutures and stitches on the struts 305-3. Exemplary methods of performing the sixth operation 561 and attaching the tissue bumpers 161 to the frame 102 are disclosed in U.S. Application Number 63/300,281, filed January 18, 2022 (Attorney Docket Number A0003308US01) that is incorporated by reference herein in its entirety.

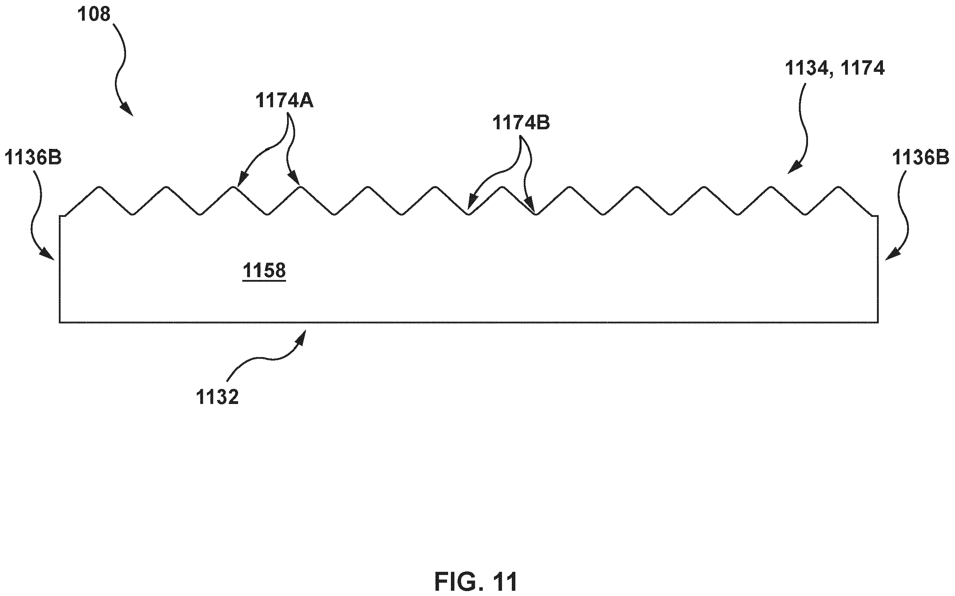

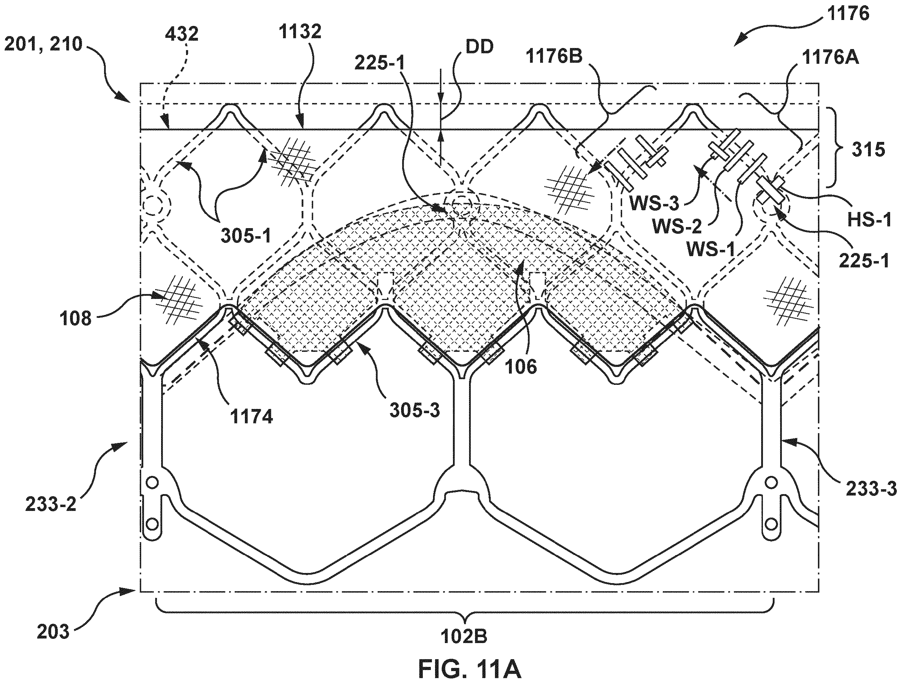

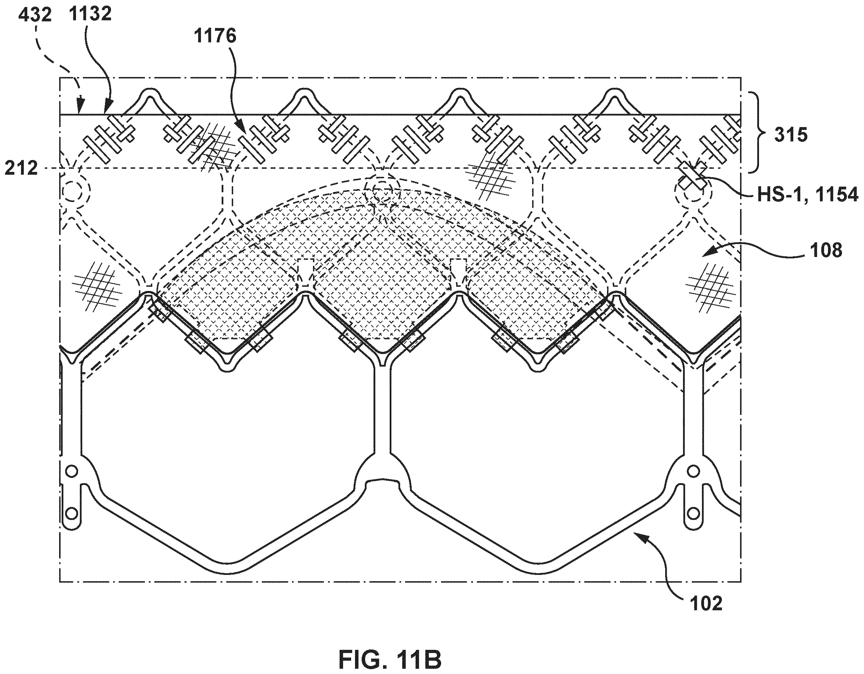

In a seventh operation 563 of the method 550, the outer wrap 108 is attached proximate an inflow end 1132 thereof to the frame 102 of the transcatheter heart valve prosthesis 100. FIG. 11 shows a flat, as-cut outer wrap 108. The outer wrap 108 is a piece of graft material having a substantially rectangular shape that is sized to be wrapped around the inflow section 211 of the frame 102 and secured thereto. The outer wrap 108 includes the inflow edge or end 1132, and outflow edge or end 1134, a first side edge 1136A, a second side edge 1136B, and an exterior surface 1158. The inflow edge 1132 is a straight edge, and the first and second side edges 1136A, 1136B extend from opposing ends of the inflow edge 1132 to respective opposing ends of the outflow edge 1134. The outflow edge 1134 has a zigzag pattern 1174 that includes a plurality of peaks 1174A with a plurality of valleys 1174B, with each valley 1174B being disposed between a pair of adj acent peaks 1174A. The zigzag pattern 1174 of the outflow edge 1134 substantially corresponds to or mirrors a position or pattern of the struts 305-3 in the third strut row 319 of the frame 102, such that the outflow edge 1134 of the outer wrap 108 may align with the struts 305-3 in the third strut row 319, which will be explained in more detail below. In accordance with embodiments hereof, an outer wrap 108 is a material selected to prevent or prohibit paravalvular leakage through the valve prosthesis and may be a laser cut piece of material that is composed of a woven or knitted fabric, pericardial tissue, or a polymer material that prevents or prohibits paravalvular leakage. In an embodiment, an outer wrap 108 is made of a material that has a loop pile on an exterior surface 1158 thereof, *see,* for *e.g.,* FIG. 1, that aids in prevention of paravalvular leakage (PVL) by locally reducing flow in gaps between the valve and the native anatomy. In addition, the loop pile material has a high surface area which promotes tissue ingrowth, enhancing biologic processes to help close PVL pathways.

For performing the seventh operation, the frame 102 is flipped upside down, *i.e.,* with its inflow end 201 above its outflow end 203, as shown for the second segment 102B of the frame 102 in FIGS. 11A and 11B. In a first step, the outer wrap 108 is wrapped around an exterior of the frame 102 and the straight inflow edge 1132 of the outer wrap 108 is aligned with the straight inflow edge 432 of the inner skirt 106, the zigzag outflow edge 1174 of the outer wrap 108 is substantially aligned with the struts 305-3 in the third strut row 319 of the frame, and the first and second side edges 1136A, 1136B are brought together and aligned with each other. The inner skirt 106 was previously tacked to the frame 102 in the first operation 551 to cover the struts 305-1 in the first strut row 315 of the inflow section 211 except for leaving exposed the inflow crowns or nodes 0 in the inflow node row 210, as previously noted above, such that the inflow edge 432 of the inner skirt 106 ends at a defined distance DD from the inflow node row 210 as shown in FIGS. 6A and 11A. Similarly, the outer wrap 108 covers the exterior of the struts 305-1 in the first strut row 315 of the inflow section 211 except for leaving exposed the inflow crowns or nodes 0 in the inflow node row 210, such that the inflow edge 1132 of the outer wrap 108 is also spaced a defined distance DD from the inflow node row 210 to be co-extensible with the inflow edge 432 of the inner skirt 106, as shown in FIGS. 11A and 11B. With the outer wrap 108 so positioned, a needle (not shown) and a suture (not shown) are used to stitch the first side edge 1136A to the second side edge 1136B, and thereby create a side seam (not shown) of the outer wrap 108 that is, preferably, aligned with the side seam 106A of the inner skirt 106.

A wrap-frame attachment pattern 1176 is then sewn with a needle (not shown) and a suture (not shown) to attach the outer wrap 108 proximate its inflow edge 1132 to the frame 102 along portions of the struts 305-1 in the first strut row 315. Prior to starting the wrap-frame attachment pattern 1176, the prior made tack stitches 262 (not shown in FIGS. 11A and 11B) located at each marker 225-1 are located and removed from the leaflet under the current portion of the outer wrap 108 to be stitched, such as from the leaflet 107B (not shown) with reference to the frame segment 102B shown in FIGS. 11A and 11B. The wrap-frame attachment pattern 1176 is started by the suture being used to create a first horizontal stitch HS-1 at the marker 225-1 that is aligned with the third commissure post 233-3. The suture of the first horizontal stitch HS-1 should extend through only a material of the outer wrap 108 to attach the outer wrap 108 to the strut 305-1 at the marker 225-1, as shown in FIGS. 11A and 11B. A first segment 1176A of the wrap-frame attachment pattern 1176 is then created by sewing first, second and third whip stitches WS-1, WS-2, WS-3 to connect the strut 305-1 to the outer wrap 108, with the third whip stitch WS-3 being a locking stitch disposed proximate the outflow edge 1132 of the outer wrap 108. Thereafter, a second segment 1176B of the wrap-frame attachment pattern 1176 is then created by sewing first, second and third whip stitches WS-1, WS-2, WS-3 to connect a next strut 305-1 to the outer wrap 108, with the first whip stitch WS-1 being a locking stitch disposed proximate the inflow edge 1132 of the outer wrap 108. The first and second segments 1176A, 1176B are repeated around the outer wrap 108 until the initial horizontal stitch HS-1 is reached, and then a knot is made to tie off the suture and thereby complete the wrap-frame attachment pattern 1176 and the seventh operation 563. In accordance with embodiments hereof, the locking stitches utilized in the seventh operation assure that the outer wrap 108 is securely connected to the bottom row of inflow struts to keep the outer wrap 108 in place during crimping of the transcatheter heart valve prosthesis 100 and also to minimize inward protrusion of the outer wrap 108 when crimped.

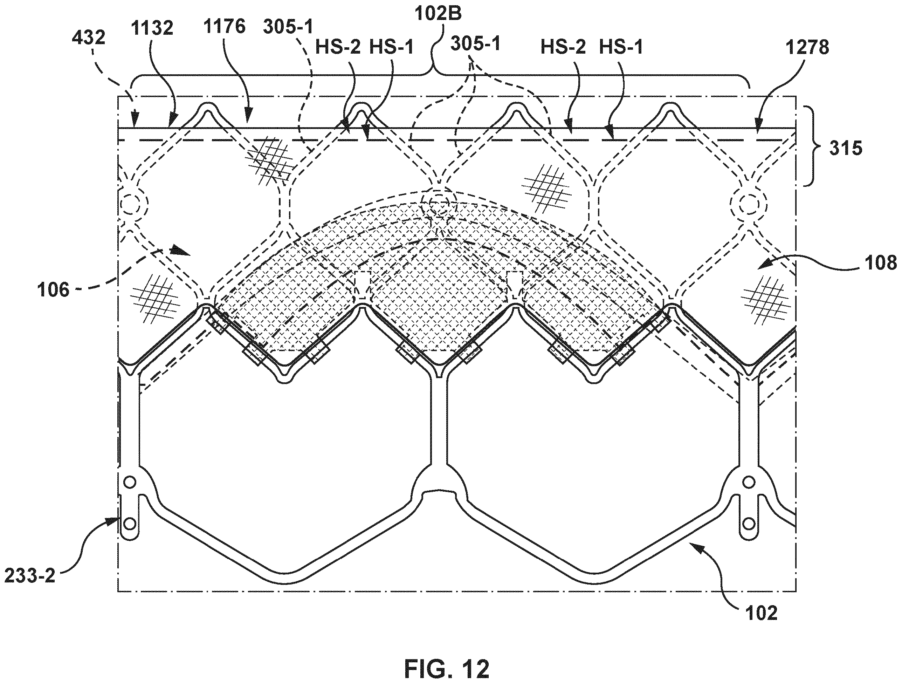

In an eighth operation 565 of the method 550 with the frame 102 still flipped upside down as in the seventh operation 563, the outer wrap 108 is sewn to the inner skirt 106 at the inflow end 201 of the frame 102. With reference to FIG. 12, a suture is used to sew a straight line of horizontal stitches 1278 that attaches the inner skirt 106 and the outer wrap 108 together proximate the inflow edges 432, 1132 thereof, whereby no stiches in the line of horizontal stitches 1278 are wrapped around a strut or node of the frame. The line of horizontal stitches 1278 runs in parallel to the inflow edges 432, 1132 of the inner skirt 106 and the outer wrap 108 and is spaced therefrom by approximately 1 mm. In an embodiment, a pair of horizontal stitches HS-1, HS-2 of the line of horizontal stitches 1278 is disposed between each pair of adjacent struts 305-1 in the first strut row 315 of the inflow section 211, as shown in FIG. 12. Once the line of horizontal stitches 1278 is complete, a knot is tied at the end of the suture to secure the stitches in place. In an embodiment as described herein, the inflow edges 432, 1132 of the inner skirt 106 and the outer wrap 108 are sewn or attached to each other without being attached to the frame therebetween such that when the heart valve prosthesis 100 is expanded the attached inflow edges 432, 1132 of the inner skirt 106 and the outer wrap 108 are readily slidable relative to the struts in the first strut row 315 of the frame 102, without getting caught or snagged on the exposed inflow crowns or nodes 0.

In a ninth operation 567 of the method 550, with the frame 102 returned to an upright position, the outer wrap 108 is sewn to the inner skirt 106 and the outer wrap backing 964 by a line of outflow edge stitches 1380 that follows or mirrors the zigzag pattern 1174 of the outflow edge 1134 of the outer wrap 108. The line of outflow edge stitches 1380 does not attach these pieces to the frame 102, *i.e.,* when making the outflow edge stitches the suture is not wrapped around the struts/nodes of the frame. As well, in accordance with embodiments hereof and further to the actions taken in the outer wrap backing/inner skirt 964, 106 attachment to the frame 102 of the fifth operation 559 and in the tissue bumper attachment of the sixth operation 561, the outflow edge 1134 of the outer wrap 108 is not wrapped over the top struts 305-3 of the third strut row 319, but instead the outflow edge 1134 of the outer wrap 108 is disposed to be secured in place while maintaining a gap between the top struts 305-3 and the material of the outer wrap 108. This gap allows for the tabs of the outer wrap backing 964 and the additional tissue or reinforcement material of the tissue bumpers 161 to be disposed along the third strut row 319 of the top struts 305-3 of the inflow section 211 without increasing profile when the transcatheter heart valve prosthesis 100 is in a crimped state.

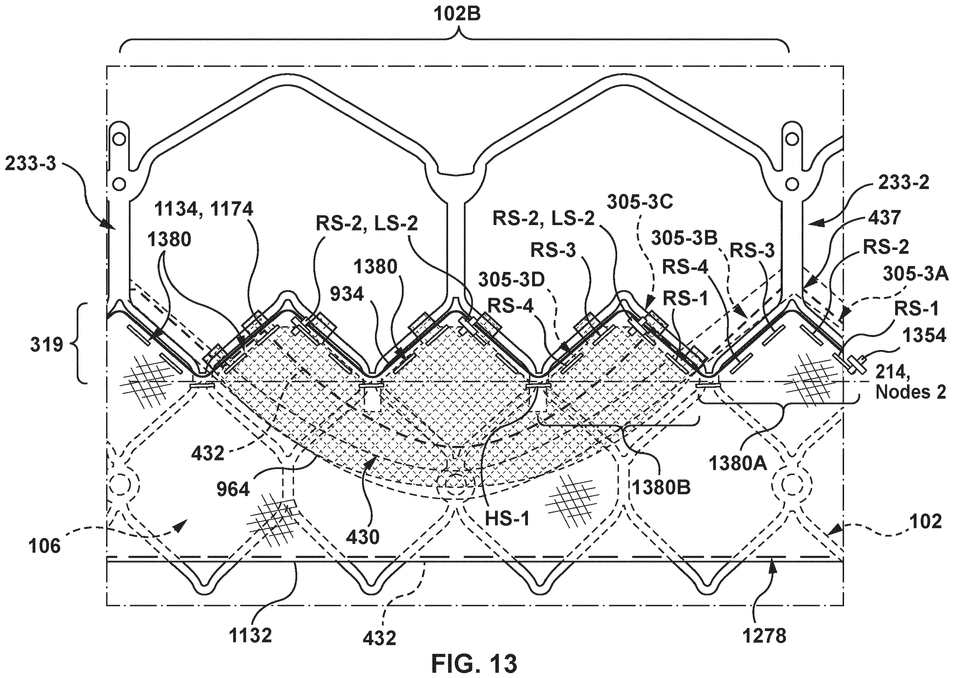

FIG. 13 illustrates a portion of the line of outflow edge stitches 1380 extending within the frame segment 102B that connects the outer wrap 108 to the inner skirt 106 in the inferior commissure area ICA of the commissure posts 233-2, 233-3 and connects the outer wrap 108 to the outer wrap backing 964 proximate the outflow edges 934, 1134 thereof above the margin of attachment area 430 of the valve-skirt assembly 422. In accordance with embodiments hereof, the outflow edge stitches 1380 directly suture to each other. The outer wrap backing 964 and the outer wrap 108 layers are in an area above the margin of attachment 430 in order to prevent leakage through the transcatheter heart valve prosthesis 100.

A needle with a suture attached thereto (not shown) is used to create a knot 1354 proximate a node 2 of the second node row 214 that starts the line of outflow edge stitches 1380 at a location below the commissure post 233-2, which is by way of example and not limitation as the line of stitches could be started under one of the other commissure posts 233-1, 233-3 without departing from the scope hereof. Next, first and second running stitches RS-1, RS-2 are sewn parallel to a strut 305-3A in the third strut row 319 and third and fourth running stitches RS-3, RS-4 are sewn parallel to a strut 305-3B in the third strut row 319. The first, second, third and fourth running stitches RS-1, RS-2, RS-3, RS-4 are sewn to attach a peak 437 of the inner skirt 106 to the outer wrap 108 proximate the outflow edge 1134 thereof. When the suture reaches the next node 2, a horizontal stitch HS-1 is made within the outer wrap 108, above/across the node 2, to end a first segment 1380A of the line of outflow edge stitches 1380, as shown in FIG. 13.

Next, first and second running stitches RS-1, RS-2 are sewn parallel to a strut 305-3C in the third strut row 319 and third and fourth running stitches RS-3, RS-4 are sewn parallel to a strut 305-3D in the third strut row 319. The first, second, third and fourth running stitches RS-1, RS-2, RS-3, RS-4 are sewn to attach the outer wrap backing 964 to the outer wrap 108 proximate the outflow edges 934, 1134 thereof above the margin of attachment area 430 of the valve-skirt assembly. In an embodiment, each second running stitch RS-2 within the second segment 1380B of the line of outflow edge stitches 1380 is a locking stich LS-2 that is proximate a node 3 in the third node row 216 and the locking stitch LS-1 is made to tightly secure the outer wrap backing 964 to the outer wrap 108 by pulling the outer wrap backing 964 flat to prevent slack or protrusions on the inside of the frame 102. When the suture reaches the next node 2 in the second node row 214, a horizontal stitch HS-1 is made only within the outer wrap 108, above/across the node 2, to end a second segment 1380B of the line of outflow edge stitches 1380, as shown in FIG. 13.

The second segment 1380B of stitches is then continued below and in parallel to the next pairs of struts 305-3C, 305-3D to attach the outer wrap backing 964 to the outer wrap 108 proximate the outflow edges 934, 1134 thereof above the margin of attachment area 430 of the valve-skirt assembly for the remainder of the frame segment 102B. Thereafter the first and second segments 1380A, 1380B are repeated in this manner within the first and third frame segments 102A, 102C until the starting knot 1354 is reached and the suture is then tied off to complete the line of outflow edge stitches 1380.

When assembled as described above, bottom curved portions of the margin of attachments 430 of the leaflets 107A, 107B, 107C of the valve-skirt assembly 422 of the transcatheter heart valve prosthesis 100 are not directly attached to the frame 102 but instead the bottom curved portions of the margin of attachments 430 of the leaflets 107A, 107B, 107C may be described as being unsupported by the frame 102. The unsupported margins of attachment (MOA) 430 below each leaflet 107A, 107B, 107C of the valve-skirt assembly 422 allow the load to be transferred from the leaflets to the skirt material to reduce the risk of leaflet tearing. Additionally, in cases of non-uniform deployment, the unsupported MOA 430 is less distorted because the inner skirt 106 can accommodate some of the slack or tension due to asymmetry.

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components.

Further disclosed herein is the subject-matter of the following clauses:
1. A transcatheter valve prosthesis (100) comprising:
   a frame (102) having an inflow section (211) and an outflow section (213), the frame being a substantially tubular structure that defines a central lumen (209) of the prosthesis;
   a valve skirt assembly (422) having a valve component (104) and an inner skirt (106), the valve-skirt assembly being disposed within the central lumen of the frame;
   an outer wrap backing (964) attached to an exterior of the inflow section of the frame;
      and
   an outer wrap (108) disposed over the outer wrap backing and attached to the frame to cover the remainder of the exterior of the inflow section of the frame.
2. The valve prosthesis of clause 1, wherein the inner skirt has a scalloped outflow edge (434) that is comprised of a plurality of cut-outs (434-1, 434-2, 434-3) and a plurality of peak regions (437-1, 437-2, 437-3).
3. The valve prosthesis of clause 2, wherein each cut-out extends between adjacent peak regions of the inner skirt, and/or wherein the outer wrap backing is a plurality of outer wrap backings with each outer wrap backing of the plurality of outer wrap backings being disposed on the exterior of the frame opposite a respective cut-out of the plurality of cut-outs of the inner skirt.
4. The valve prosthesis of clause 2, wherein each of the outer wrap backing (964) has a plurality of tabs (965A, 965B, 965C) extending from an outflow edge (934) thereof that are attached to a plurality of struts (305-3) of a top strut row (319) of the inflow section of the frame.
5. The valve prosthesis according to any of the previous clauses, wherein the valve component is comprised of a plurality of leaflets (107A, 107B, 107C) with each leaflet of the plurality of leaflets being attached to the inner skirt at a respective margin of attachment (430), and wherein bottom curved portions of the margin of attachments between the plurality of leaflets and the inner skirt are not directly attached to the frame.
6. The valve prosthesis according to any of the previous clauses, wherein the outer wrap backing and the outer wrap are made of biocompatible materials that prevent or prohibit paravalvular leakage through the frame of the valve prosthesis.
7. The valve prosthesis according to any of the previous clauses, wherein straight inflow edges of the inner skirt and the outer wrap are aligned with each and the straight inflow edges are spaced from inflow nodes of an inflow node row of the frame to leave the inflow nodes exposed, and wherein the inflow edges of the inner skirt and the outer wrap are attached to each other by a line of horizontal stitches that parallels the inflow edges without wrapping around the frame therebetween.
8. A method (550) of assembling a transcatheter heart valve prosthesis (100) comprising:
   tacking a valve-skirt assembly (422) within an interior of a frame (102), the valve skirt assembly having a valve component (104) and an inner skirt (106);
   attaching the inner skirt to the frame within a plurality of inferior commissure areas (ICA) that are located below a plurality of commissure posts (233-1, 233-2, 233-3) of the frame;
   attaching a plurality of commissures (132-1, 132-2, 132-3) of the valve component to the plurality of commissure posts of the frame;
   attaching a plurality of outer wrap backings (964) to the inner skirt with the frame situated therebetween;
   attaching the plurality of outer wrap backings (964) and the inner skirt to the frame;
   attaching tissue bumpers (161) to struts (305-3, 305-4) proximate to or within an outflow section (213) of the frame;
   attaching an outer wrap (108) and the inner skirt to the frame;
   attaching the outer wrap to the inner skirt proximate inflow edges (432, 1132) thereof;
      and
   attaching the outer wrap to the inner skirt and to the outer wrap backings proximate outflow edges (434, 934, 1134) thereof.
9. The method of clause 8, wherein each outer wrap backing of the plurality of outer wrap backings is disposed on the exterior of the frame opposite a respective cut-out of a plurality of cut-outs (434-1, 434-2, 434-3) within the outflow edge (434) of the inner skirt, and wherein attaching the plurality of outer wrap backings (964) to the inner skirt includes securing a curved outflow edge (932) of each of the outer wrap backings to the inner skirt, proximate a respective cut-out, by using a suture to create a blanket-stitch pattern (970) therebetween.
10. The method according to any of the previous clauses, wherein attaching the plurality of outer wrap backings (964) and the inner skirt (106) to the frame includes attaching a plurality of tabs (965A, 965B, 965C) that extend from the outflow edge (934) of each outer wrap backing (964) to a plurality of struts (305-3) of a top strut row (319) of an inflow section (211) of the frame.
11. The method according to any of the previous clauses, wherein attaching the outer wrap (108) and the inner skirt to the frame includes attaching the inflow edge (1132) of the outer wrap to a plurality of struts (305-1) of a bottom strut row (315) of an inflow section (211) of the frame by using a suture to create a wrap-frame attachment pattern (1176) proximate the inflow edge (1132) of the outer wrap that secures the outer wrap to the plurality of struts (305-1) of the bottom strut row (315).
12. The method of clause 11, wherein attaching the outer wrap to the inner skirt proximate inflow edges (432, 1132) thereof includes aligning the inflow edges (432, 1132) of the inner skirt (106) and the outer wrap (108) with each such that the inflow edges (432, 1132) are spaced from inflow nodes (0) of an inflow node row (210) of the frame 102 to leave the inflow nodes exposed.
13. The method of clause 12, wherein attaching the outer wrap to the inner skirt proximate inflow edges (432, 1132) thereof includes using a suture to create a line of horizontal stitches (1278) that parallels the inflow edges of the outer wrap and the inner skirt without the suture wrapping around the frame therebetween.
14. The method according to any of the previous clauses, wherein attaching the outer wrap to the inner skirt and to the outer wrap backings proximate outflow edges (434, 934, 1134) thereof includes using a suture to create a line of outflow edge stitches (1380) that parallels struts (305-3) in a third strut row (319) of the frame without the suture wrapping around the frame.
15. The method according to any of the previous clauses, wherein the valve component is comprised of a plurality of leaflets (107A, 107B, 107C) with each leaflet of the plurality of leaflets being attached to the inner skirt at a respective margin of attachment (430), and wherein bottom curved portions of the margin of attachments between the plurality of leaflets and the inner skirt are not directly attached to the frame.

## Claims

1. A transcatheter valve prosthesis (100) comprising:
a frame (102) having an inflow section (211) and an outflow section (213), the frame being a substantially tubular structure that defines a central lumen (209) of the prosthesis;
a valve skirt assembly (422) having a valve component (104) and an inner skirt (106), the valve-skirt assembly being disposed within the central lumen of the frame;
an outer wrap backing (964) attached to an exterior of the inflow section of the frame;
and
an outer wrap (108) disposed over the outer wrap backing and attached to the frame to cover the remainder of the exterior of the inflow section of the frame.

2. The valve prosthesis of claim 1, wherein the inner skirt has a scalloped outflow edge (434) that is comprised of a plurality of cut-outs (434-1, 434-2, 434-3) and a plurality of peak regions (437-1, 437-2, 437-3).

3. The valve prosthesis of claim 2, wherein each cut-out extends between adjacent peak regions of the inner skirt, and/or wherein the outer wrap backing is a plurality of outer wrap backings with each outer wrap backing of the plurality of outer wrap backings being disposed on the exterior of the frame opposite a respective cut-out of the plurality of cut-outs of the inner skirt.

4. The valve prosthesis of claim 2, wherein each of the outer wrap backing (964) has a plurality of tabs (965A, 965B, 965C) extending from an outflow edge (934) thereof that are attached to a plurality of struts (305-3) of a top strut row (319) of the inflow section of the frame.

5. The valve prosthesis according to any of the previous claims, wherein the valve component is comprised of a plurality of leaflets (107A, 107B, 107C) with each leaflet of the plurality of leaflets being attached to the inner skirt at a respective margin of attachment (430), and wherein bottom curved portions of the margin of attachments between the plurality of leaflets and the inner skirt are not directly attached to the frame.

6. The valve prosthesis according to any of the previous claims, wherein the outer wrap backing and the outer wrap are made of biocompatible materials that prevent or prohibit paravalvular leakage through the frame of the valve prosthesis.

7. The valve prosthesis according to any of the previous claims, wherein straight inflow edges of the inner skirt and the outer wrap are aligned with each and the straight inflow edges are spaced from inflow nodes of an inflow node row of the frame to leave the inflow nodes exposed, and wherein the inflow edges of the inner skirt and the outer wrap are attached to each other by a line of horizontal stitches that parallels the inflow edges without wrapping around the frame therebetween.

8. A method (550) of assembling a transcatheter heart valve prosthesis (100) comprising:
tacking a valve-skirt assembly (422) within an interior of a frame (102), the valve skirt assembly having a valve component (104) and an inner skirt (106);
attaching the inner skirt to the frame within a plurality of inferior commissure areas (ICA) that are located below a plurality of commissure posts (233-1, 233-2, 233-3) of the frame;
attaching a plurality of commissures (132-1, 132-2, 132-3) of the valve component to the plurality of commissure posts of the frame;
attaching a plurality of outer wrap backings (964) to the inner skirt with the frame situated therebetween;
attaching the plurality of outer wrap backings (964) and the inner skirt to the frame;
attaching tissue bumpers (161) to struts (305-3, 305-4) proximate to or within an outflow section (213) of the frame;
attaching an outer wrap (108) and the inner skirt to the frame;
attaching the outer wrap to the inner skirt proximate inflow edges (432, 1132) thereof;
and
attaching the outer wrap to the inner skirt and to the outer wrap backings proximate outflow edges (434, 934, 1134) thereof.

9. The method of claim 8, wherein each outer wrap backing of the plurality of outer wrap backings is disposed on the exterior of the frame opposite a respective cut-out of a plurality of cut-outs (434-1, 434-2, 434-3) within the outflow edge (434) of the inner skirt, and wherein attaching the plurality of outer wrap backings (964) to the inner skirt includes securing a curved outflow edge (932) of each of the outer wrap backings to the inner skirt, proximate a respective cut-out, by using a suture to create a blanket-stitch pattern (970) therebetween.

10. The method according to any of the previous claims, wherein attaching the plurality of outer wrap backings (964) and the inner skirt (106) to the frame includes attaching a plurality of tabs (965A, 965B, 965C) that extend from the outflow edge (934) of each outer wrap backing (964) to a plurality of struts (305-3) of a top strut row (319) of an inflow section (211) of the frame.

11. The method according to any of the previous claims, wherein attaching the outer wrap (108) and the inner skirt to the frame includes attaching the inflow edge (1132) of the outer wrap to a plurality of struts (305-1) of a bottom strut row (315) of an inflow section (211) of the frame by using a suture to create a wrap-frame attachment pattern (1176) proximate the inflow edge (1132) of the outer wrap that secures the outer wrap to the plurality of struts (305-1) of the bottom strut row (315).

12. The method of claim 11, wherein attaching the outer wrap to the inner skirt proximate inflow edges (432, 1132) thereof includes aligning the inflow edges (432, 1132) of the inner skirt (106) and the outer wrap (108) with each such that the inflow edges (432, 1132) are spaced from inflow nodes (0) of an inflow node row (210) of the frame 102 to leave the inflow nodes exposed.

13. The method of claim 12, wherein attaching the outer wrap to the inner skirt proximate inflow edges (432, 1132) thereof includes using a suture to create a line of horizontal stitches (1278) that parallels the inflow edges of the outer wrap and the inner skirt without the suture wrapping around the frame therebetween.

14. The method according to any of the previous claims, wherein attaching the outer wrap to the inner skirt and to the outer wrap backings proximate outflow edges (434, 934, 1134) thereof includes using a suture to create a line of outflow edge stitches (1380) that parallels struts (305-3) in a third strut row (319) of the frame without the suture wrapping around the frame.

15. The method according to any of the previous claims, wherein the valve component is comprised of a plurality of leaflets (107A, 107B, 107C) with each leaflet of the plurality of leaflets being attached to the inner skirt at a respective margin of attachment (430), and wherein bottom curved portions of the margin of attachments between the plurality of leaflets and the inner skirt are not directly attached to the frame.
